# EUROPEAN PATENT SPECIFICATION

(11) **EP 1 892 240 B1**
(45) Date of publication and mention of the grant of the patent: **18.04.2012**
(21) Application number: 07114529.6
(22) Date of filing: 17.08.2007
(51) Int. Cl.: C07D 209/64, C09B 69/00

(54) **Benzoisoindole derivative, pigment composition comprising the same and colored composition**
Benzoisoindol-Derivat, Pigmentzusammensetzung damit und gefärbte Zusammensetzung
Dérivé de benzoisoindole, composition de pigment comportant celui-ci et composition colorée

(30) Priority: 18.08.2006 JP 2006222826
(43) Date of publication of application: 27.02.2008
(73) Proprietor: TOYO INK MFG. CO., LTD., Tokyo (JP)
(72) Inventor: Goto, Shoko, Chuo-ku Tokyo (JP); Kimura, Shuuichi, Chuo-ku Tokyo (JP); Takayama, Masakazu, Chuo-ku Tokyo (JP); Yanai, Hiroyuki, Chuo-ku Tokyo (JP)
(74) Representative: TBK

(56) References cited:
- WO-A1-2004/076456
- US-A1- 2005 120 911
- DATABASE WPI Derwent Publications Ltd., London, GB; AN 2007-382660, XP002458593 & JP 2007 084659 A (TOYO INK MFG) 05 April 2007

## Description

The present invention relates to a pigment composition that allows a fine dispersion of pigment particles to be easily obtained and maintained in a stable state. More particularly, the invention relates to a pigment composition suitably used in, for example, printing inks, paints, resin coloring agents, ink-jet inks or color filter inks, and to a colored composition using the same.

Printing inks and paints exhibit their high coloring ability by finely dispersing the pigment, and thereby printed matters and coatings are endowed with clear color tone and glossiness. Further, it is necessary to highly pulverize the pigment particles for obtaining high level of clearness in offset inks, gravure inks and paints, in particular in ink-jet inks and color filter inks. Examples of the method for pulverizing the pigment particles that is widely used today include a solvent salt milling method and a dry milling method. However, when the pigment is simply charged together with usually used milling aids such as inorganic salts and solvents, growth of pigment particles simultaneously proceeds during the pulverization process due to the heat generated by pulverization and by the action of solvents added as dispersion aids. In addition, since the pigment particles become unstable by applying a long-term mechanical force, changes such as crystal transition may occur to sometimes fail to stably and sufficiently pulverize the particles even by spending much time and energy. In particular, since the particle size of the pigment is liable to be irregular by using the dry milling method only, the particle size is regulated by solvent treatment. However, regulation of the particle size is difficult by the solvent treatment since finer pigment particles are more liable to cause crystal growth.

When the pigment is further pulverized, the ink and paint often come to have high viscosity due to enhanced cohesive force between the pigment particles. In addition, when producing the dispersion, the high viscosity makes it difficult to retrieve the product from a dispersion machine or to transfer the product to a tank from the dispersion machine while, in the worse case, the product is not available due to gelling during storage.

For solving these problems, a pigment dispersant has been used for improving affinity between the pigment and a vehicle in order to stabilize the dispersed material, and various pigment dispersants have been disclosed (see, for example, Jpn. Pat. Appln. KOKAI Publication Nos. 3-26767 and 6-316676). Examples of the dispersant include pigment derivatives prepared by introducing a functional group such as an acidic group, a basic group or phthalimidomethyl group into an organic pigment, and resin type dispersants prepared by introducing an acidic group or a basic group into a part of an acrylic polymer or a polyester resin. These dispersants are used singly or in combination and are found effective. Some of these dispersants also have an effect of preventing crystal growth of the pigment.

The state of the art reports on several pigment compositions. As such, the JP 2005-084659 discloses a pigment composition containing a pigment, an acidic functional group containing organic dye derivatives, alkali-soluble resins having an amino group and/or quaternary ammonium salts and organic solvents. The US-A-2005/0120911 discloses a polymeric dispersant constituted by a polyether which is end-caped with a benzoindole derivative. Further, the WO-A-2004/076456 discloses a variety of organic compounds, processes for their manufacture, and their use. As such, this document relates to an essential solvent-free process for the preparation of the alkali-metal salts of diketopyrrolopyrrole compounds, and their use as latent pigments.

However, the effect of preventing crystal growth of the pigment of a conventional pigment dispersant is not always sufficient for stably obtaining highly pulverized pigment particles developed for attaining high clearness in printing inks and paints, in particular ink-jet inks and color filter inks.

The above-described pigment dispersants do not always afford sufficiently low viscosity and high storage stability to inks and paints that contain highly pulverized pigments. In addition, much energy is required for obtaining high glossiness and transparency by disintegrating tightly aggregated fine pigment particles into color-developing particles.

When different kinds of pigments are mixed and used, stability of the pigments as an ink is impaired, and color separation due to aggregation and precipitation of pigments occur to consequently cause color shading and remarkable decrease in coloring ability in the color-developed matters. Furthermore, the surface of the coating film of the color-developed matters may be impaired by decreased glossiness and leveling insufficiency.

Accordingly, there is a need for a pigment composition which has a high effect of stabilizing the pigment particles and of preventing crystal growth of the pigment particles and which enables stably and highly pulverized pigment particles to be obtained, and a pigment composition which has high dispersibility, excellent viscosity characteristics and storage stability even in ink-jet inks and color filter inks prepared by dispersing highly pulverized pigment particles.

A first object of the present invention is to provide a compound that can suppress the crystal growth of pigments, stabilize fine pigment particles, and lower the viscosity when it is combines with pigments.

A second object of the present invention is to provide a pigment composition in which pigment particles are stably and highly pulverized while crystal growth of the pigment is suppressed.

Further, a third object of the invention is to provide a colored composition that is suitable for use in printing inks and paints, in particular in ink-jet inks and color filter inks where fine dispersion of the pigment particles is required, and that has low viscosity and is remarkably excellent in stability with time.

According to a first object of the present invention, there is provided a benzoisoindole derivative represented by Formula (1): 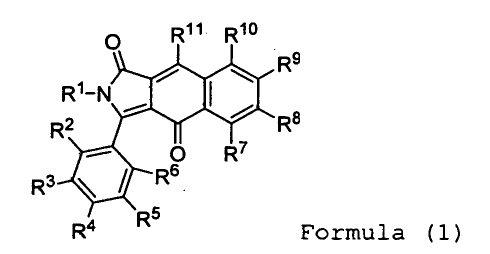 where:
R¹ denotes a hydrogen atom, an alkyl group that may have one or more substituents, or an acyl group that may have one or more substituents;
R², R³, R⁵, R⁶, R⁷, R⁸, R⁹ and R¹⁰ each independently denote a hydrogen atom, an alkyl group that may have one or more substituents, a phenyl group, a halogen atom, a cyano group, an alkoxy group that may have one or more substituents, or -NR¹²R¹³, where R¹² and R¹³ each independently denote a hydrogen atom or an alkyl group that may have one or more substituents, or R¹² and R¹³ may be bonded together to form a heterocyclic ring that may further contain a nitrogen, oxygen or sulfur atom;
R⁴ denotes a hydrogen atom, an alkyl group that may have one or more substituents, an acyl group that may have one or more substituents, or a substituent represented by Formula (2):

-Y-(Z)ₖ (2)

where Y denotes a direct bond, -O-, -S(O)ₘ- (where m denotes an integer of 0 to 3), -CO-, -NR¹⁴-, -CONR¹⁴-, -SO₂NR¹⁴, -NR¹⁴CO-, NR¹⁴SO₂- (where R¹⁴ denotes a hydrogen atom, an alkyl group or a hydroxylalkyl group), a linear or branched alkylene having 1 to 12 carbon atoms, or a benzene residue or triazine residue that may be substituted with an alkyl group, an amino group, a nitro group, a hydroxyl group, an alkoxy group or a halogen atom, or a linking group formed of two or more of these linking groups; Z denotes a hydrogen atom, a halogen atom, an alkyl group that may have one or more substituents, an aryl group that may have one or more substituents, a phthalimidomethyl group that may have one or more substituents, -NR¹⁵R¹⁶, -SO₃·M/n or -COO·M/n, where R¹⁵ and R¹⁶ each independently denote a hydrogen atom, an alkyl group that may have one or more substituents, an alkenyl group that may have one or more substituents or a phenyl group that may have one or more substituents, or R¹⁵ and R¹⁶ may be bonded together to form a heterocyclic ring that may further contain a nitrogen, oxygen or sulfur atom and that may have one or more substituents; M denotes a hydrogen ion, a mono- to trivalent metal ion or an ammonium ion at least one hydrogen atom of which is substituted with an alkyl group; n denotes valence of M; and k denotes an integer of 1 or 2; and

R¹¹ denotes a hydrogen atom, a halogen atom, a nitro group, a cyano group, a hydroxyl group, an alkoxy group that may have one or more substituents, an aryloxy group that may have one or more substituents, -S(O)ₘR¹⁷, -NR¹⁸R¹⁹ or -N₂⁺X⁻, where m is an integer of from 0 to 3, R¹⁷ denotes a hydrogen atom, an alkyl group that may have one or more substituents, an aryl group that may have one or more substituents, or a halogen atom; R¹⁸ and R¹⁹ each independently denote a hydrogen atom or an alkyl group that may have one or more substituents, or R¹⁸ and R¹⁹ may be bonded together to form a heterocyclic ring that may further contain a nitrogen, oxygen or sulfur atom; and X denotes a halogen atom.

According to a second aspect of the present invention, there is provided a pigment composition comprises a pigment and a benzoisoindole derivative represented by Formula (1): 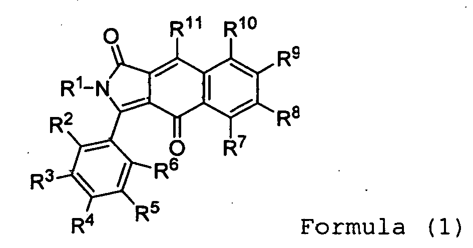 where:
R¹ denotes a hydrogen atom, an alkyl group that may have one or more substituents, or an acyl group that may have one or more substituents;
R², R³, R⁵, R⁶, R⁷, R⁸, R⁹ and R¹⁰ each independently denote a hydrogen atom, an alkyl group that may have one or more substituents, a phenyl group, a halogen atom, a cyano group, an alkoxy group that may have one or more substituents, or -NR¹²R¹³, where R¹² and R¹³ each independently denote a hydrogen atom or an alkyl group that may have one or more substituents, or R¹² and R¹³ may be bonded together to form a heterocyclic ring that may further contain a nitrogen, oxygen or sulfur atom;
R⁴ denotes a hydrogen atom, an alkyl group that may have one or more substituents, an acyl group that may have one or more substituents, or a substituent represented by Formula (2):

   -Y-(Z)ₖ (2)
where Y denotes a direct bond, -O-, -S(O)ₘ- (where m denotes an integer of 0 to 3), -CO-, -NR¹⁴-, -CONR¹⁴-, -SO₂NR¹⁴, -NR¹⁴CO-, NR¹⁴SO₂- (where R¹⁴ denotes a hydrogen atom, an alkyl group or a hydroxylalkyl group), a linear or branched alkylene having 1 to 12 carbon atoms, or a benzene residue or triazine residue that may be substituted with an alkyl group, an amino group, a nitro group, a hydroxyl group, an alkoxy group or a halogen atom, or a linking group formed of two or more of these linking groups; Z denotes a hydrogen atom, a halogen atom, an alkyl group that may have one or more substituents, an aryl group that may have one or more substituents, a phthalimidomethyl group that may have one or more substituents, -NR¹⁵R¹⁶, -SO₃·M/n or -COO.M/n, where R¹⁵ and R¹⁶ each independently denote a hydrogen atom, an alkyl group that may have one or more substituents, an alkenyl group that may have one or more substituents or a phenyl group that may have one or more substituents, or R¹⁵ and R¹⁶ may be bonded together to form a heterocyclic ring that may further contain a nitrogen, oxygen or sulfur atom and that may have one or more substituents; M denotes a hydrogen ion, a mono- to trivalent metal ion or an ammonium ion at least one hydrogen atom of which is substituted with an alkyl group; n denotes valence of M; and k denotes an integer of 1 or 2; and

R¹¹ denotes a hydrogen atom, a halogen atom, a nitro group, a cyano group, a hydroxyl group, an alkoxy group that may have one or more substituents, an aryloxy group that may have one or more substituents, -S(O)ₘR¹⁷, -NR¹⁸R¹⁹ or -N₂⁺X⁻, where m is an integer of from 0 to 3, R¹⁷ denotes a hydrogen atom, an alkyl group that may have one or more substituents, an aryl group that may have one or more substituents, or a halogen atom; R¹⁸ and R¹⁹ each independently denote a hydrogen atom or an alkyl group that may have one or more substituents, or R¹⁸ and R¹⁹ may be bonded together to form a heterocyclic ring that may further contain a nitrogen, oxygen or sulfur atom; and X denotes a halogen atom.

The pigment composition may further contain a pigment derivative, preferably at least one pigment derivative selected from the group consisting of pigment derivatives comprising a diketopyrrolopyrrole, a quinacridone, thiazine-indigo or anthraquinone pigment residue having a basic group, an acidic group or a phthalimidomethyl group introduced thereinto.

Further, according to the present invention, there is provided a colored composition comprising the pigment composition above and a pigment carrier.

The invention can be more fully understood from the following detailed description when taken in conjunction with the accompanying drawings, in which:
The single figure shows an ORTEP figure obtained from single crystal X-ray structure analysis of Compound (3) described below.

The benzoisoindole derivative of the present invention is represented by Formula (1) above, and the pigment composition of the invention contains a pigment and a benzoisoindole derivative represented by Formula (1) above.

In Formula (1):
R¹ denotes a hydrogen atom, an alkyl group that may have one or more substituents, or an acyl group that may have one or more substituents;
R², R³, R⁵, R⁶, R⁷, R⁸, R⁹ and R¹⁰ each independently denote a hydrogen atom, an alkyl group that may have one or more substituents, a phenyl group, a halogen atom, a cyano group, an alkoxy group that may have one or more substituents, or -NR¹²R¹³, where R¹² and R¹³ each independently denote a hydrogen atom or an alkyl group that may have one or more substituents, or R¹² and R¹³ may be bonded together to form a heterocyclic ring that may further contain a nitrogen, oxygen or sulfur atom;
R⁴ denotes a hydrogen atom, an alkyl group that may have one or more substituents, an acyl group that may have one or more substituents, or a substituent represented by Formula (2):

   -Y-(Z)ₖ (2)
where Y denotes a direct bond, -O-, -S(O)ₘ- (where m denotes an integer of from 0 to 3), -CO-, -NR¹⁴-, -CONR¹⁴-, -SO₂NR¹⁴ -NR¹⁴CO-, NR¹⁴SO₂- (where R¹⁴ denotes a hydrogen atom, an alkyl group or a hydroxylalkyl group), a linear or branched alkylene having 1 to 12 carbon atoms, or a benzene residue or triazine residue that may be substituted with an alkyl group, an amino group, a nitro group, a hydroxyl group, an alkoxy group or a halogen atom, or a linking group formed of two or more of these linking groups; Z denotes a hydrogen atom, a halogen atom, an alkyl group that may have one or more substituents, an aryl group that may have one or more substituents, a phthalimidomethyl group that may have one or more substituents, -NR¹⁵R¹⁶, -SO₃·M/n or -COO·M/n, where R¹⁵ and R¹⁶ each independently denote a hydrogen atom, an alkyl group that may have one or more substituents, an alkenyl group that may have one or more substituents or a phenyl group that may have one or more substituents, or R¹⁵ and R¹⁶ may be bonded together to form a heterocyclic ring that may further contain a nitrogen, oxygen or sulfur atom and that may have one or more substituents; M denotes a hydrogen ion, a mono- to trivalent metal ion or an ammonium ion at least one hydrogen atom of which is substituted with an alkyl group; n denotes valence of M; and k denotes an integer of 1 or 2; and

R¹¹ denotes a hydrogen atom, a halogen atom, a nitro group, a cyano group, a hydroxyl group, an alkoxy group that may have one or more substituents, an aryloxy group that may have one or more substituents, -S(O)ₘR¹⁷, -NR¹⁸R¹⁹ or -N₂⁺X⁻, where m is an integer of from 0 to 3, R¹⁷ denotes a hydrogen atom, an alkyl group that may have one or more substituents, an aryl group that may have one or more substituents, or a halogen atom; R¹⁸ and R¹⁹ each independently denote a hydrogen atom or an alkyl group that may have one or more substituents, or R¹⁸ and R¹⁹ may be bonded together to form a heterocyclic ring that may further contain a nitrogen, oxygen or sulfur atom; and X denotes a halogen atom.

According to a preferred embodiment of the present invention, in Formula (1):
R¹ denotes a hydrogen atom;
R², R³, R⁵, R⁶, R⁷, R⁸, R⁹ and R¹⁰ each independently denote a hydrogen atom, an alkyl group, a phenyl group, a halogen atom, a cyano group, an alkoxy group, or -NR¹²R¹³, where R¹² and R¹³ each independently denote a hydrogen atom or an alkyl group having 1 to 12 carbon atoms, or R¹² and R¹³ may be bonded together to form a heterocyclic ring that may further contain a nitrogen, oxygen or sulfur atom;
R⁴ denotes a hydrogen atom, an alkyl group that may have one or more substituents, an acyl group that may have one or more substituents, or a substituent represented by Formula (2) above; and
R¹¹ denotes a hydrogen atom, a halogen atom, a nitro group, a cyano group, a hydroxyl group, an alkoxy group, an aryloxy group, -S(O)ₘR¹⁷, -NR¹⁸R¹⁹ or -N₂⁺X⁻, where m is an integer of from 0 to 3, R¹⁷ denotes a hydrogen atom, an alkyl group, an aryl group, or a halogen atom; R¹⁸ and R¹⁹ each independently denote a hydrogen atom or an alkyl group having 1 to 12 carbon atoms, or R¹⁸ and R¹⁹ may be bonded together to form a heterocyclic ring that may further contain a nitrogen, oxygen or sulfur atom; and X denotes a halogen atom.
In this preferred embodiment, R¹¹ more preferably denotes a hydrogen atom or -NR¹⁸R¹⁹.

The alkyl group that may have one or more substituents is preferably an alkyl group having 1 to 30 carbon atoms, and examples of the alkyl group include methyl group, ethyl group, propyl group, butyl group, hexyl group, octyl group, decyl group, dodecyl group, octadecyl group, isopropyl group, isobutyl group, sec-butyl group, tert-butyl group, 1-ethylpentyl group, cyclopentyl group, cyclohexyl group, trifluoromethyl group, 2-ethylhexyl group, phenacyl group, 1-naphthoylmethyl group, 2-naphthoylmethyl group, 4-methylsulfanylphenacyl group, 4-phenylsulfanylphenacyl group, 4-dimethylaminophenacyl group, 4-cyanophenacyl group, 4-methylphenacyl group, 2-methylphenacyl group, 3-fluorophenacyl group, 3-trifluoromethylphenacyl group and 3-nitrophenacyl group.

The acyl group that may have one or more substituents is preferably an acyl group having 2 to 20 carbon atoms, and examples of the acyl group include acetyl group, propanoyl group, butanoyl group, trifluoromethylcarbonyl group, pentanoyl group, benzoyl group, 1-naphthoyl group, 2-naphthoyl group, 4-methylsulfanylbenzoyl group, 4-phenylsulfanylbenzoyl group, 4-dimethylaminobenzoyl group, 4-diethylaminobenzoyl group, 2-chlorobenzoyl group, 2-methylbenzoyl group, 2-methoxybenzoyl group, 2-butoxybenzoyl group, 3-chlorobenzoyl group, 3-trifluoromethylbenzoyl group, 3-cyanobenzoyl group, 3-nitrobenzoyl group, 4-fluorobenzoyl group, 4-cyanobenzoyl group and 4-methoxybenzoyl group.

Examples of the halogen atom include fluorine atom, chlorine atom, bromine atom and iodine atom.

The alkoxy group that may have one or more substituents is preferably an alkoxy group having 1 to 30 carbon atoms, and examples of the alkoxy group include methoxy group, ethoxy group, propyloxy group, isopropyloxy group, butoxy group, isobutoxy group, sec-butoxy group, tert-butoxy group, pentyloxy group, isopentyloxy group, hexyloxy group, heptyloxy group, octyloxy group, 2-ethylhexyloxy group, decyloxy group, dodecyloxy group, octadecyloxy group, ethoxycarbonylmethyl group, 2-ethylhexyloxycarbonylmethyloxy group, aminocarbonylmethyloxy group, N,N-dibutylaminocarbonylmethyloxy group, N-methylaminocarbonylmethyloxy group, N-ethylaminocarbonylmethyloxy group, N-octylaminocarbonylmethyloxy group, N-methyl-N-benzylaminocarbonylmethyloxy group, benzyloxy group and cyanomethyloxy group.

The aryl group that may have one or more substituents is preferably an aryl group having 6 to 30 carbon atoms, and examples of the aryl group include phenyl group, biphenyl group, 1-naphthyl group, 2-naphthyl group, 9-anthryl group, 9-phenanthryl group, 1-pyrenyl group, 5-naphthacenyl group, 1-indenyl group, 2-azulenyl group, 9-fluorenyl group, terphenyl group, quarter phenyl group, o-, m- and p-tolyl groups, xylyl group, o-, m- and p-cumenyl groups, mesityl group, pentalenyl group, binaphthalenyl group, ternaphthalenyl group, quarter naphthalenyl group, heptalenyl group, biphenylenyl group, indacenyl group, fluoranthenyl group, acenaphthylenyl group, aceanthrylenyl group, phenalenyl group, fluorenyl group, anthryl group, bianthracenyl group, teranthracenyl group, quarter anthracenyl group, anthraquinolyl group, phenanthryl group, triphenylenyl group, pyrenyl group, chrysenyl group, naphthacenyl group, preiadenyl group, picenyl group, perylenyl group, pentaphenyl group, pentacenyl group, tetraphenylenyl group, hexaphenyl group, hexacenyl group, rubicenyl group, coronenyl group, trinaphthylenyl group, heptaphenyl group, heptacenyl group, pyranthrenyl group and ovalenyl group.

The aryloxy group that may have one or more substituents is preferably an aryloxy group having 6 to 30 carbon atoms, and examples of the aryloxy group include phenyloxy group, 1-naphthyloxy group, 2-naphthyloxy group, 2-chlorophenyloxy group, 2-methylphenyloxy group, 2-methoxyphenyloxy group, 2-butoxyphenyloxy group, 3-chlorophenyloxy group, 3-trifluoromethylphenyloxy group, 3-cyanophenyloxy group, 3-nitrophenyloxy group, 4-fluorophenyloxy group, 4-cyanophenyloxy group, 4-methoxyphenyloxy group, 4-dimethylaminophenyloxy group, 4-methylsulfanylphenyloxy group and 4-phenylsulfanylphenyloxy group.

Hydrogen atoms in the alkyl group, acyl group, alkoxy group, aryl group and aryloxy group may be further substituted with other substituents.

Examples of such substituents include halogen atoms such as fluorine, chlorine, bromine and iodine atoms; alkoxy groups such as methoxy, ethoxy and tert-butoxy groups; aryloxy groups such as phenoxy and p-tolyloxy groups; alkoxycarbonyl groups such as methoxycarbonyl, butoxycarbonyl and phenoxycarbonyl groups; acyloxy groups such as acetoxy, propyonyloxy and benzoyloxy groups; acyl groups such as acetyl, benzoyl, isobutylyl, acryloyl, methacryloyl and methoxyallyl groups; alkyl sulfanyl groups such as methyl sulfanyl and tert-butyl sulfanyl groups; aryl sulfanyl groups such as phenyl sulfanyl and p-tolyl sulfanyl groups; alkylamino groups such as methylamino and cyclohexylamino groups; dialkylamino groups such as dimethylamino, diethylamino, morpholino and piperidino groups; arylamino groups such as phenylamino and p-tolylamino groups; alkyl groups such as methyl, ethyl, tert-butyl and dodecyl groups; aryl groups such as phenyl, p-tolyl, xylyl, cumenyl, naphthyl, anthryl and phenanthryl groups; and heterocyclic groups such as furyl and thienyl groups; as well as hydroxyl group, carboxyl group, formyl group, mercapto group, sulfo group, mesyl group, p-toluenesulfonyl group, amino group, nitro group, cyano group, trifluoromethyl group, trichloromethyl group, trimethylsilyl group, phosphinyl group, phosphono group, trimethylammonium group, dimethylsulfonium group and triphenylphenacyl phosphonium group.

R¹² and R¹³, or R¹⁸ and R¹⁹ may be bonded together to form a heterocyclic ring, which may further contain nitrogen, oxygen or sulfur atoms.

Examples of the phthalimidomethyl group that may have one or more substituents include phthalimidomethyl group, 4-nitrophthalimidomethyl group, 4-chlorophthalimidomethyl group, tetrachlorophthalimidomethyl group and (4,6-bis(phthalimidomethylamino)-1,3,5-triazin)-2-ylaminomethyl group.

Examples of mono- to trivalent metal ions include sodium ion, potassium ion, calcium ion, strontium ion, barium ion and aluminum ion.

Examples of ammonium ions at least one hydrogen atom of which is substituted with an alkyl group include octyl ammonium ion, dodecyl ammonium ion, hexadecyl ammonium ion, octadecyl ammonium ion, dimethyldidecyl ammonium ion, dimethyldidodecyl ammonium ion, dimethyldioctadecyl ammonium ion, trimethyldodecyl ammonium ion and trimethyloctadecyl ammonium ion.

Among them, the benzoisoindole derivatives, which may be able to facilitate obtaining inks and paints that exhibit excellent dispersibility in a wide variety of resins while having high crystallization preventive effect as well as excellent non-aggregation, non-crystallization, good viscosity characteristics, glossiness of the coating film, good visibility and storage stability, are those in which R⁴ is represented by Formula (3) or (4) below:

-Y-(NR¹⁵R¹⁶) ₖ (3)

In the formula, Y, R¹⁵, R¹⁶, and k are as defined with reference to Formula (2) above.

Examples of the substituent represented by Formula (3) include sulfamoyl group, methylamino group, ethylamino group, propylamnio group, butylamino group, hexylamino group, octylamino group, dodecylamino group, octadecylamino group, dimethylamino group, diethylamino group, dibutylamino group, dimethylaminopropylamino group, diethylaminopropylamino group, diethylaminoethylamino group, dibutylaminopropylamino group, piperidinomethyl group, dimethylaminomethyl group, diethylaminomethyl group, dibutylaminomethyl group, dimethylaminoethyloxy group, dimethylaminoethylthio group, diethylaminoethylthio group, diethylaminoethylsulfinyl group, (4,6-bis(diethylaminopropylamino)-1,3,5-triazin)-2-yl group, dimethylaminopropylaminosulfonyl group, diethylaminopropylaminosulfonyl group, dibutylaminopropylaminosulfonylgroup, morpholinoethylaminosulfonyl group, 4-aminophenylaminosulfonyl group, piperidinopropylaminosulfonyl group, 4-methylpiperazinopropylaminosulfonyl group, dimethylaminopropylaminocarbonyl group, 4-(diethylaminopropylaminocarbonyl)phenylaminocarbonyl group, dimethylaminomethylcarbonylaminomethyl group, diethylaminopropylaminomethylcarbonylaminomethyl group and dibutylaminopropylaminomethylcarbonylaminomethyl group.

-Y-(-SO₃·M/n or -COO·M/n)ₖ (4)

In the formula, Y, M, n and k are as defined with reference to Formula (2) above.

Examples of the substituent represented by Formula (4) include sulfo group, sodium sulfonato group, calcium sulfonato group, strontium sulfonato group, barium sulfonato group, aluminum sulfonato group, 4-(aluminumsulfonato)phenylcarbamoylmethyl group, dodecylammoniosulfonato group, octadecylammoniosulfonato group, trimethyloctadecylammoniosulfonato group, dimethyldidecylammoniosulfonato group, carboxyl group, 2-ammonium carboxylato-5-nitrobenzamidemethyl group and 4-carboxyphenylaminocarbonyl group.

Specific examples of the benzoisoindole derivatives represented by Formula (1), i.e., Compounds (1)-(112), are shown in Table 1 below. In Table 1, Me denotes a methyl group, Et denotes an ethyl group, n-Pr denotes a normal propyl group, i-Pr denotes a 2-propyl group, Bu denotes a butyl group, Hex denotes a hexyl group, Tol denotes a para-tolyl group, Ac denotes an acetyl group, Bz denotes a benzoyl group, and Ph denotes a phenyl group.

**Table 1**

| | | | |
|---|---|---|---|
| Comp. (1) | 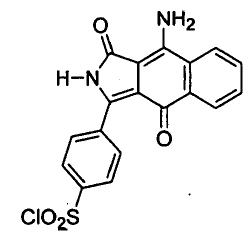 | Comp. (2) | 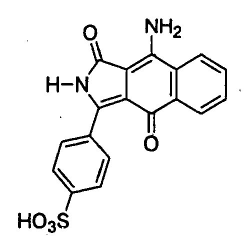 |
| Comp. (3) | 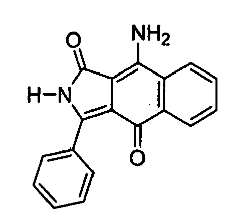 | Comp. (4) | 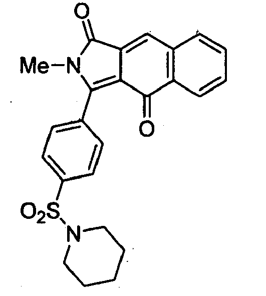 |
| Comp. (5) | 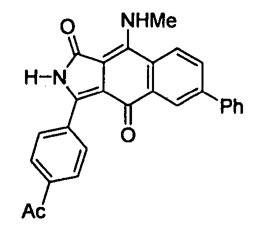 | Comp. (6) | 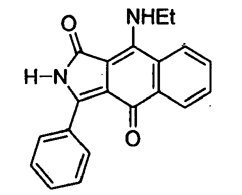 |
| Comp. (7) | 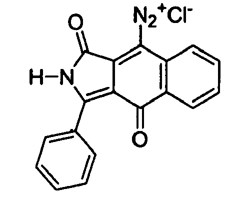 | Comp. (8) | 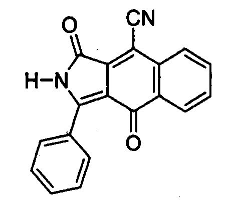 |
| Comp. (9) | 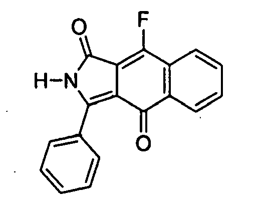 | Comp. (10) | 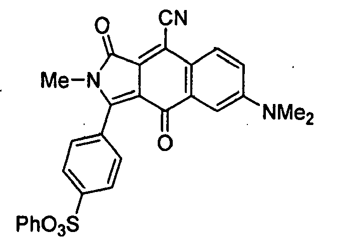 |
| Comp. (11) | 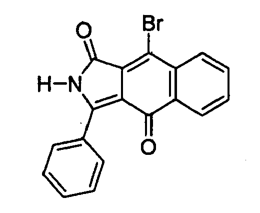 | Comp. (12) | 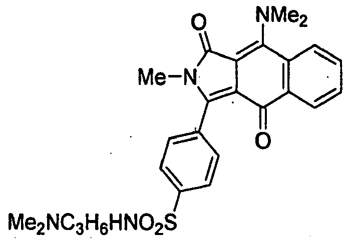 |
| Comp. (13) | 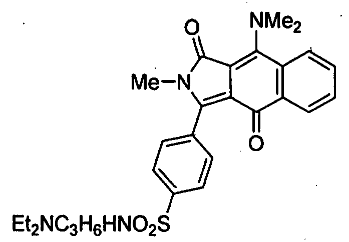 | Comp. (14) | 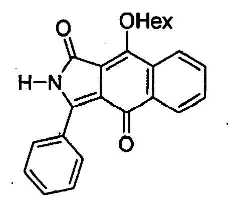 |
| Comp. (15) | 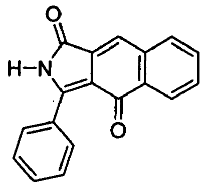 | Comp. (16) | 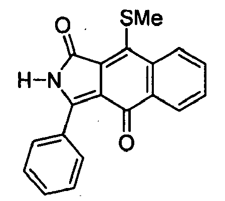 |
| Comp. (17) | 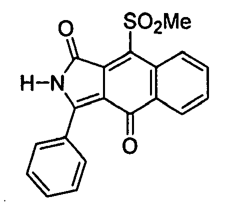 | Comp. (18) | 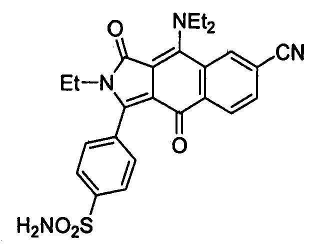 |
| Comp. (19) | 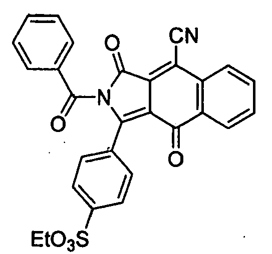 | Comp. (20) | 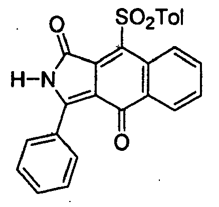 |
| Comp. (21) | 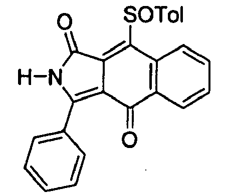 | Comp. (22) | 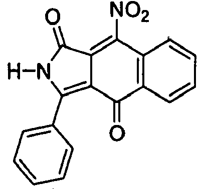 |
| Comp. (23) | 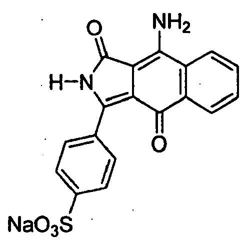 | Comp. (24) | 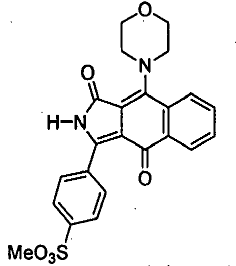 |
| Comp. (25) | 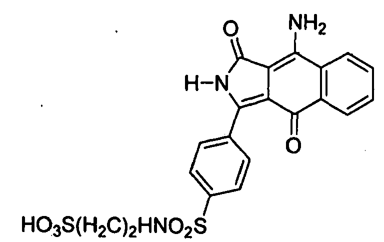 | Comp. (26) | 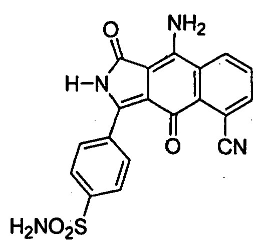 |
| Comp. (27) | 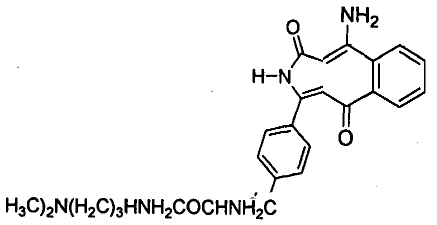 | Comp. (28) | 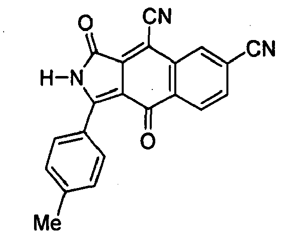 |
| Comp. (29) | 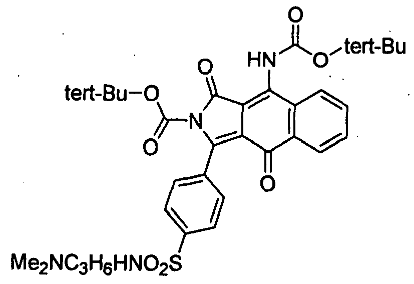 | Comp. (30) | 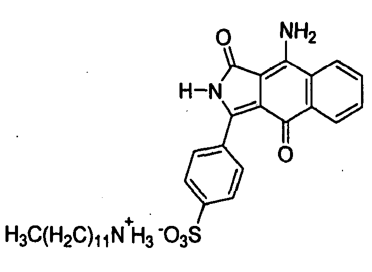 |
| Comp. (31) | 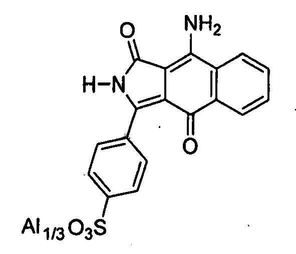 | Comp. (32) | 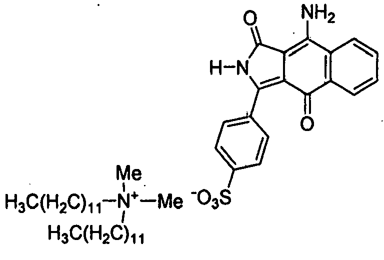 |
| Comp. (33) | 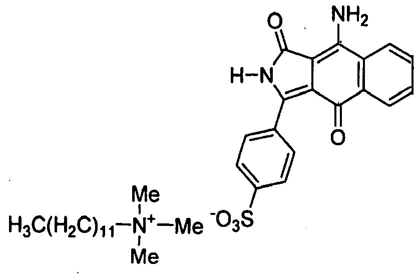 | Comp. (34) | 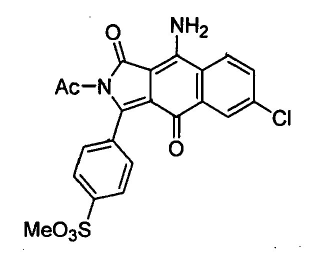 |
| Comp. (35) | 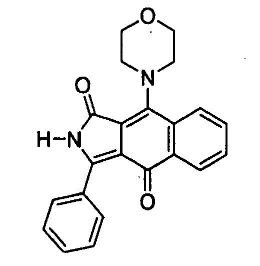 | Comp. (36) | 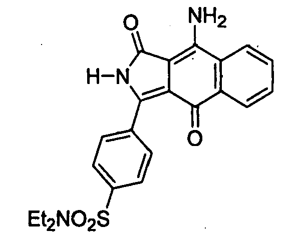 |
| Comp. (37) | 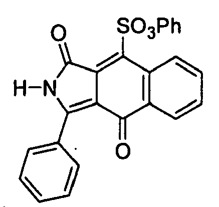 | Comp. (38) | 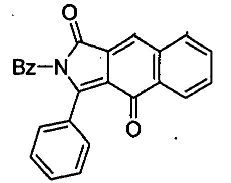 |
| Comp. (39) | 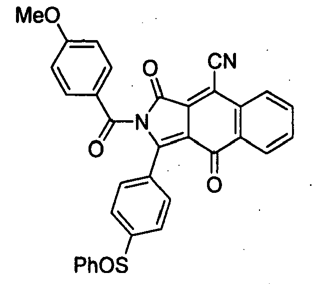 | Comp. (40) | 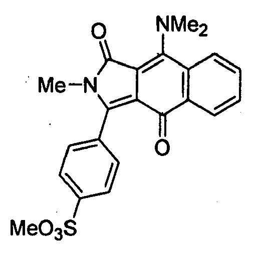 |
| Comp. (41) | 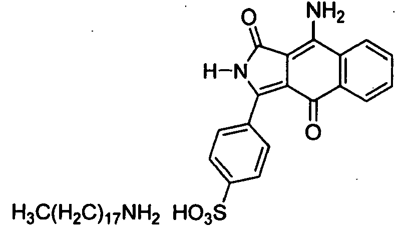 | Comp. (42) | 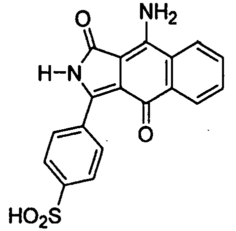 |
| Comp. (43) | 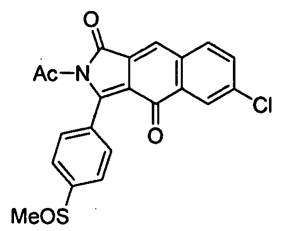 | Comp. (44) | 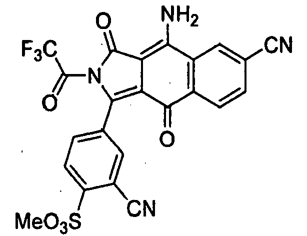 |
| Comp. (45) | 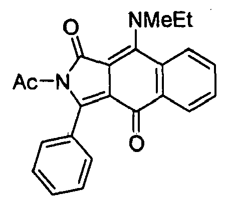 | Comp. (46) | 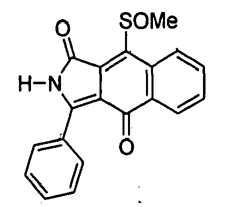 |
| Comp. (47) | 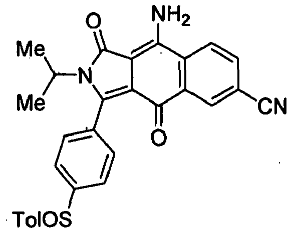 | Comp. (48) | 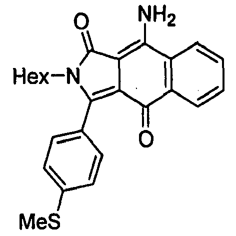 |
| Comp. (49) | 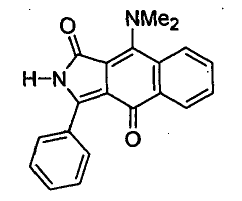 | Comp. (50) | 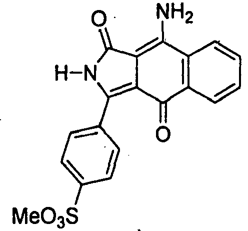 |
| Comp. (51) | 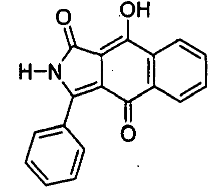 | Comp. (52) | 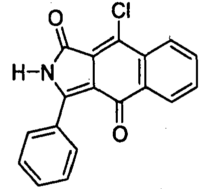 |
| Comp. (53) | 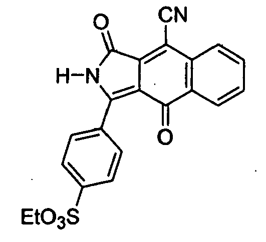 | Comp. (54) | 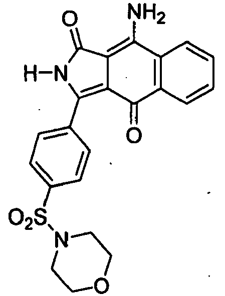 |
| Comp. (55) | 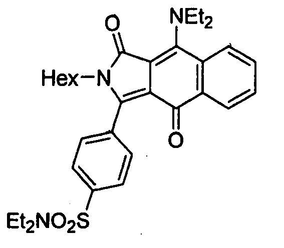 | Comp. (56) | 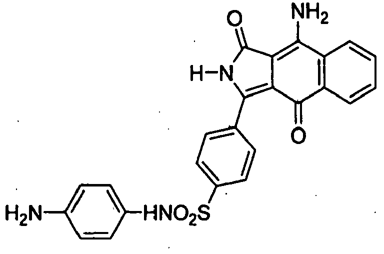 |
| Comp. (57) | 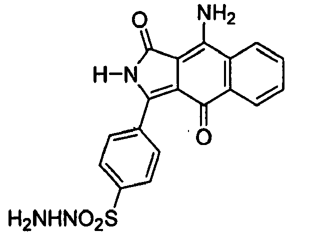 | Comp. (58) | 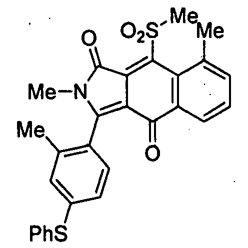 |
| Comp. (59) | 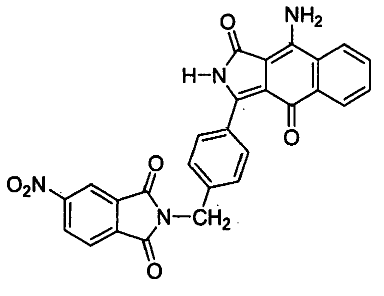 | Comp. (60) | 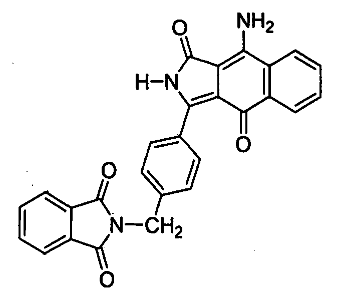 |
| Comp. (61) | 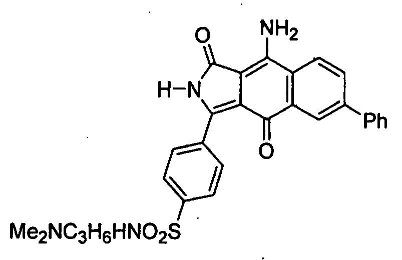 | Comp. (62) | 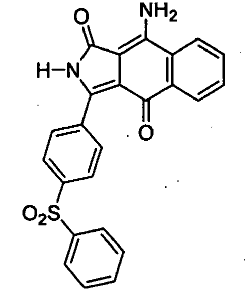 |
| Comp. (63) | 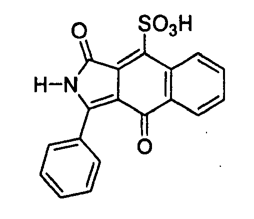 | Comp. (64) | 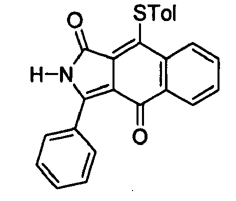 |
| Comp. (65) | 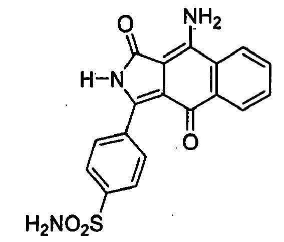 | comp. (66) | 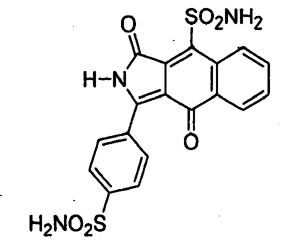 |
| Comp. (67) | 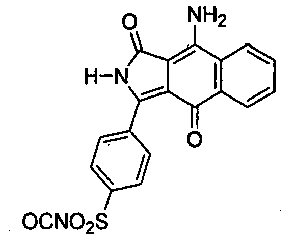 | Comp. (68) | 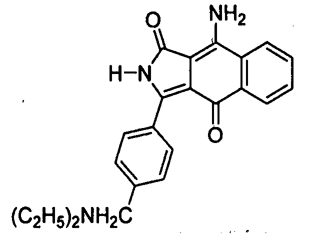 |
| Comp. (69) | 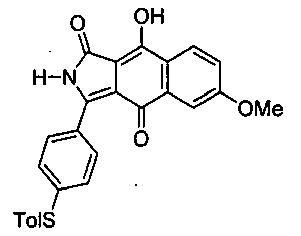 | Comp. (70) | 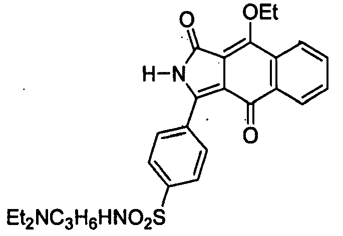 |
| Comp. (71) | 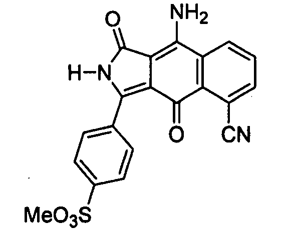 | Comp. (72) | 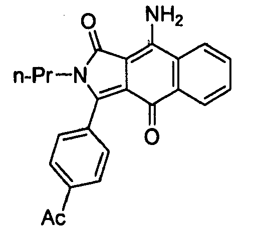 |
| Comp. (73) | 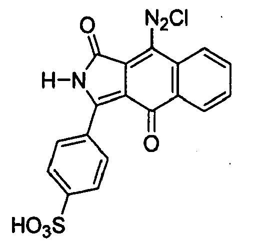 | Comp. (74) | 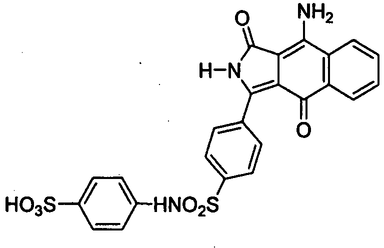 |
| Comp. (75) | 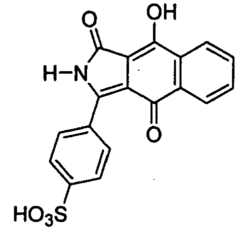 | Comp. (76) | 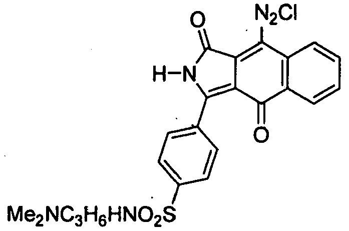 |
| Comp. (77) | 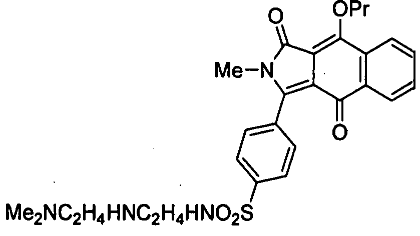 | Comp. (78) | 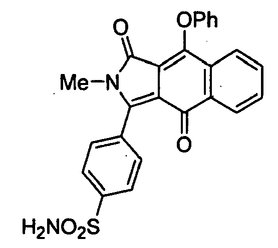 |
| Comp. (79) | 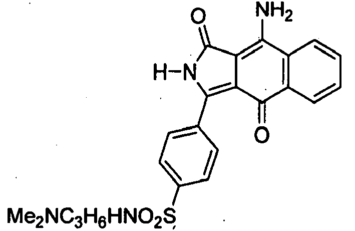 | Comp. (80) | 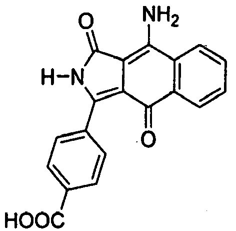 |
| Comp. (81) | 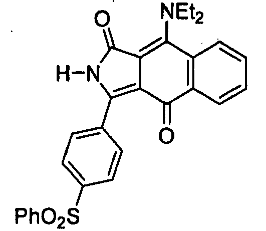 | Comp. (82) | 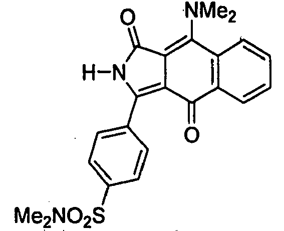 |
| Comp. (83) | 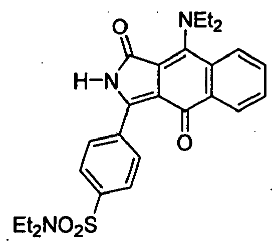 | Comp. (84) | 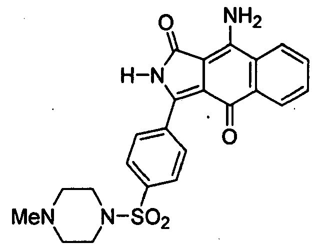 |
| Comp. (85) | 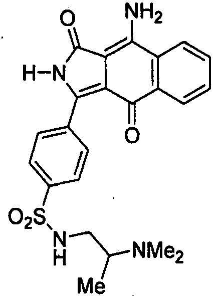 | Comp. (86) | 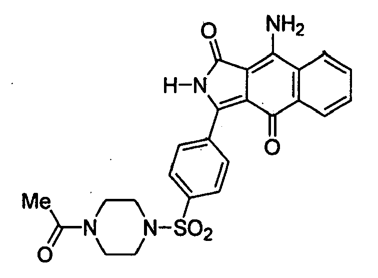 |
| Comp. (87) | 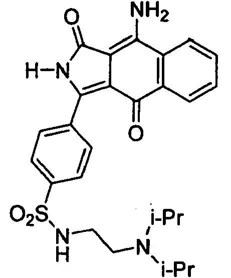 | Comp. (88) | 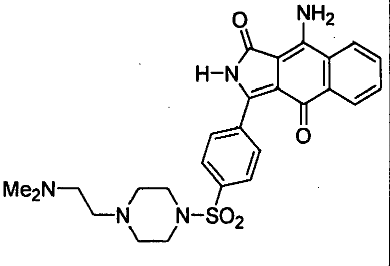 |
| Comp. (89) | 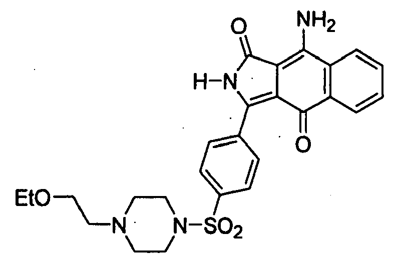 | Comp. (90) | 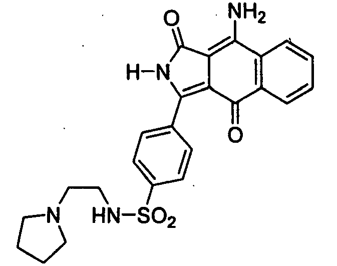 |
| Comp. (91) | 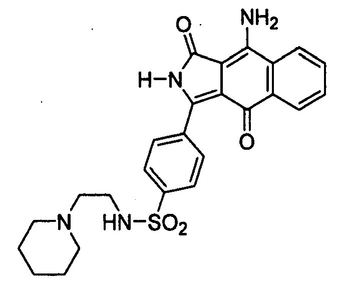 | Comp. (92) | 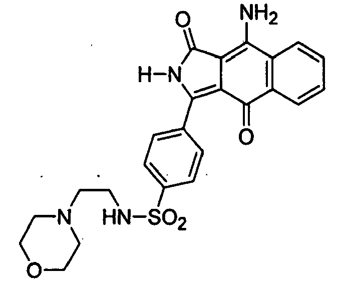 |
| Comp. (93) | 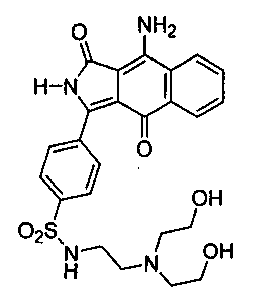 | Comp. (94) | 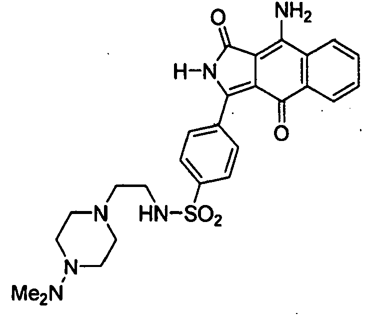 |
| Comp. (95) | 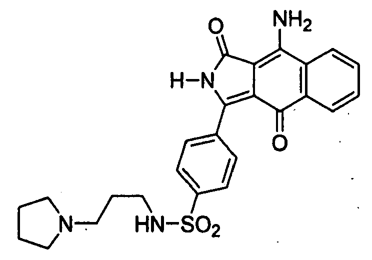 | Comp. (96) | 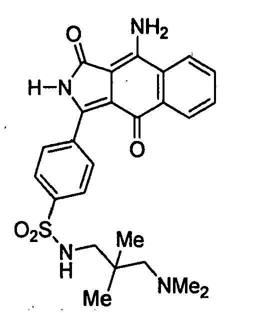 |
| Comp. (97) | 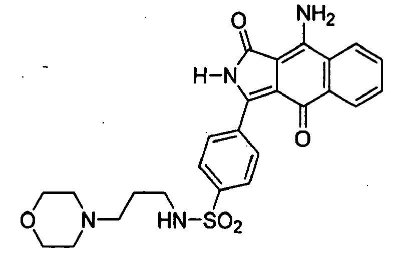 | Comp. (98) | 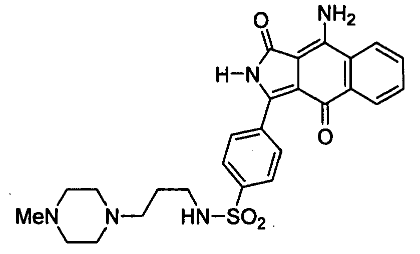 |
| Comp. (99) | 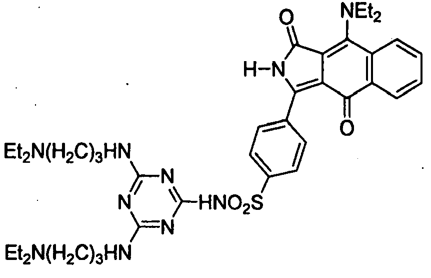 | Comp. (100) | 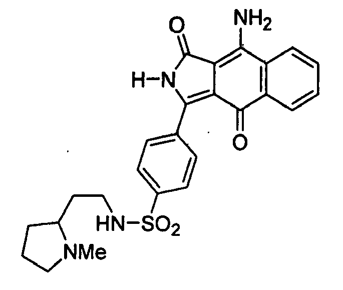 |
| Comp. (101) | 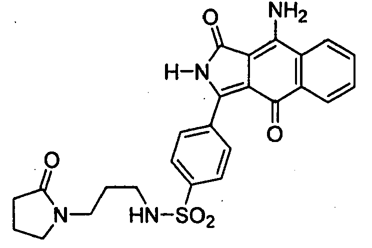 | Comp. (102) | 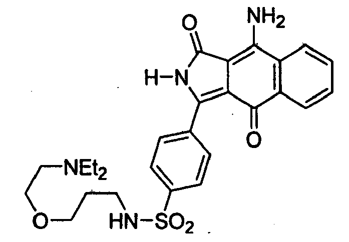 |
| Comp. (103) | 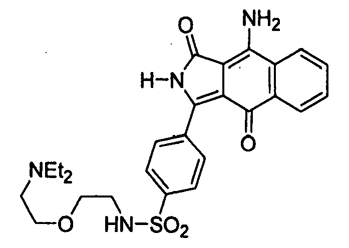 | Comp. (104) | 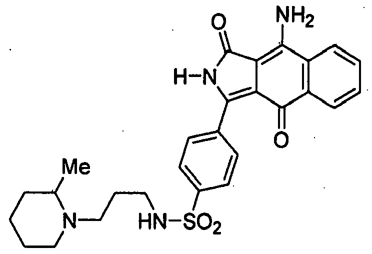 |
| Comp (105) | 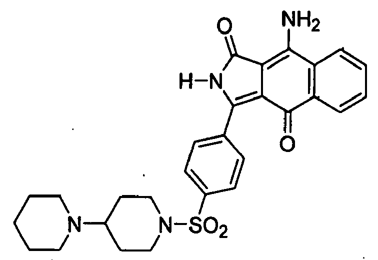 | Comp. (106) | 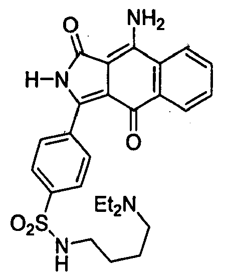 |
| Comp. (107) | 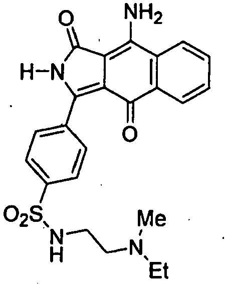 | Comp. (108) | 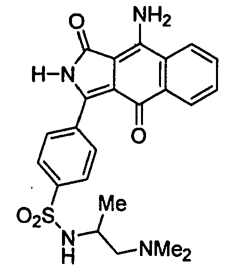 |
| Comp. (109) | 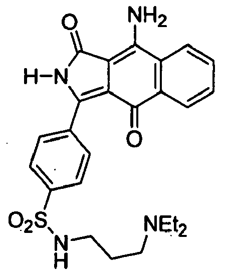 | Comp. (110) | 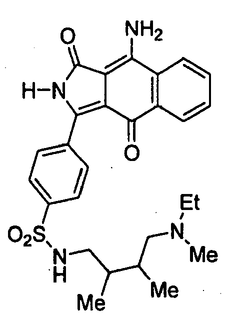 |
| Comp. (111) | 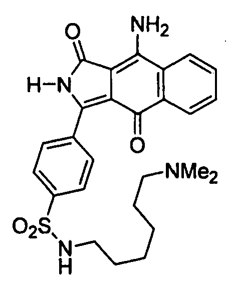 | Comp. (112) | 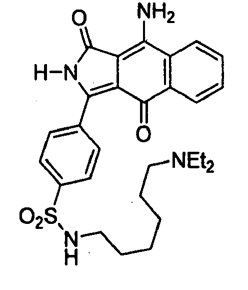 |

The compound in which R¹ denotes a hydrogen atom may have a skeleton represented by Formula (5) as a tautomer:

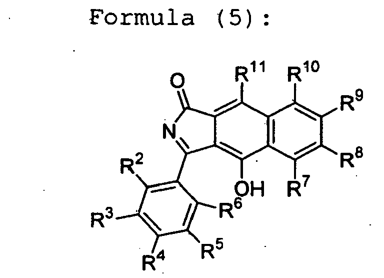

A variety of commercially available organic or inorganic pigments may be used as the pigment contained in the pigment composition of the invention. Examples of the organic pigment include azo, anthanthrone, anthrapyrimidine, anthraquinone, isoindolinone, isoindoline, indanthrone, quinacridone, quinophthalone, dioxazine, diketopyrrolopyrrole, thiazine-indigo, thioindigo, pyranthrone, phthalocyanine, flavanthrone, perynone, perylene and benzimidazolone pigments. Examples of the inorganic pigment include carbon black, titanium oxide, lead yellow, cadmium yellow, cadmium red, iron oxide red, iron oxide black, zinc oxide, prussian blue and ultramarine. These pigments may be used in combination.

The effect of preventing crystal growth of pigments and of stabilizing particles of pigments, and the effect on non-aggregation, non-crystallization and fluidity of the pigment are large with respect to the pigments having chemical structures similar to the benzoisoindole derivatives. Since the benzoisoindole derivatives develop red color, the derivatives are preferably used with the pigments having hues in the range of orange through red to rouge. Accordingly, it is particularly preferable to use thiazine-indigo red pigment, diketopyrrolopyrrole red pigment, quinacridone red pigment or anthraquinone red pigment.

By mechanically kneading a mixture of a pigment and the benzoisoindole derivative in the presence of a water-soluble inorganic salt, or of a water-soluble inorganic salt and a solvent, a pigment composition according to the invention having fine and uniform grains may be readily obtained due to the effect of preventing crystal growth of the pigment and the effect of stabilizing the particles. A kneader, a two-roll mill, a three-roll mill, a ball mill, an attritor or a sand mill may be used as the kneading machine, but the machine is not limited thereto. A coarse pigment may be used as the pigment. The proportion of the pigment and the benzoisoindole derivative is preferably from 0.1 to 30 parts by weight, more preferably from 0.5 to 25 parts by weight of benzoisoindole derivative, relative to 100 parts by weight of the pigment in order to attain the desired effect.

Examples of the water-soluble inorganic salt used in the mechanical kneading include, but are not limited to, sodium chloride, potassium chloride and anhydrous sodium sulfate. The water-soluble inorganic salt is used preferably in the range of 100 to 2,000 parts by weight, more preferably in the range of 300 to 1,000 parts by weight, relative to 100 parts by weight of the pigment. While the solvent used in the mechanical kneading is not particularly limited, high boiling point solvents are preferable in terms of safety since the temperature increases during the kneading process where the solvent is liable to be evaporated. Examples of the solvent include 2-(methoxymethoxy)ethanol, 2-butoxyethanol, 2-(isopentyloxy)ethanol, 2-(hexyloxy)ethanol, diethyleneglycol, diethyleneglycol monomethylether, diethyleneglycol monoethylether, diethyleneglycol monobutylether, triethyleneglycol, triethyleneglycol monomethylether, liquid polyethyleneglycol, 1-methoxy-2-propanol, 1-ethoxy-2-propanol, dipropyleneglycol, dipropyleneglycol monomethylether, dipropyleneglycol monoethylether and low molecular weight polypropyleneglycol. The solvent is used preferably in the range of 10 to 1,000 parts by weight, more preferably 50 to 500 parts by weight, relative to 100 parts by weight of the pigment.

Of the benzoisoindole derivatives represented by Formula (1), those having a basic group or an acidic group may give a viscosity lowering effect even if they are added when the pigment particles are being pulverized, but higher effect may be obtained when the benzoisoindole derivative is added to, and mixed with the pulverized pigment as a powder state. Further, the viscosity can be effectively lowered by mechanically mixing the benzoisoindole derivative having a basic or acidic group with the pigment by using a dissolver, a high speed mixer, a homomixer, a kneader, a roll mill, an attritor, a sand mill or various pulverizers; by allowing the pigment dispersant of the invention to precipitate on the surface of the pigment by adding a solution containing the pigment dispersant of the invention to a suspension of the pigment in water or in an organic solvent; or by dissolving the organic pigment and pigment dispersant together in a solvent having a high dissolving power such as sulfuric acid, followed by coprecipitation by diluting the solution with a poor solvent such as water. The structure of the benzoisoindole derivative added when the pigment is pulverized may differ from the structure of the benzoisoindole derivative added after pulverizing the pigment.

The benzoisoindole derivative contained in the pigment composition of the invention exhibits crystal growth suppressing effect, particle stabilizing effect and viscosity lowering effect in a wide range of structures, and these effects of the benzoisoindole derivative are particularly high for thiazine-indigo red pigments, diketopyrrolopyrrole red pigments, quinacridone red pigments or anthraquinone red pigments. However, since none of the commercially available pigments have the corresponding skeleton, replacing a part of the benzoisoindole derivative with a pigment derivative having a similar or the same chemical structure as the pigment may give a good synergic effect depending on the uses where, e.g., high clearness is required.

The pigment derivative as used herein refers to a pigment having a pigment residue into which a basic group, an acidic group or a phthalimidomethyl group is introduced. Examples of the pigment residue include azo pigment residues such as azo, disazo and polyazo residues; phthalocyanine pigment residue; anthraquinone pigment residues such as diaminodianthraquinone, anthrapyrimidine, flavanthrone, antoanthrone, indanthrone, pyranthrone and violanthrone residues; quinacridone pigment residues; quinophthalone pigment residues; dioxazine pigment residues; diketopyrrolopyrrole pigment residues; perynone pigment residues; perylene pigment residues; thioindigo pigment residues; thiazine-indigo pigment residues; isoindoline pigment residues; isoindolinone pigment residues; quinophthalone pigment residues; threne pigment residues; and pigment residues of metal complex pigments.

The pigment residue having a chemical structure similar to the benzoisoindole derivative has a large effect on non-aggregation, non-crystallization and flowability as in the case of the pigment, and pigment derivatives prepared by introducing the substituent into diketopyrrolopyrrole pigment residues, quinacridone pigment residues, thiazine-indigo pigment residues or anthraquinone pigment residues may be preferably used together.

The substituent constituting the pigment derivative is a basic group, an acidic group or a phthalimidemethyl group.

Examples of the basic group include methylamino group, ethylamino group, propylamino group, butylamino group, hexylamino group, octylamino group, dodecylamino group, octadecylamino group, deimethylamino group, diethylamino group, dibutylamino group, dimethylaminopropylamino group, diethylaminopropylamino group, diethylaminoethylamino group, dibutylaminopropylamino group, piperidinomethyl group, dimethylaminomethyl group, diethylaminomethyl group, dibutylaminomethyl group, dimethylaminoethyloxy group, dimethylaminoethylthio group, (4,6-bis(diethylaminopropylamino)-1,3,5-triazin)-2-yl group, dimethylaminopropylaminosulfonyl group, diethylaminopropylaminosulfonyl group, dibutylaminopropylaminosulfonyl group, morpholinoethylaminosulfonyl group, 4-aminophenylaminosulfonyl group, dimethylaminopropylaminocarbonyl group, 4-(diethylaminopropylaminocarbonyl)phenylaminocarbonyl group, dimethylaminomethylcarbonylaminomethyl group, diethylaminopropylaminomethylcarbonylaminomethyl group and dibutylaminopropylaminomethylcarbonylaminomethyl group.

Examples of the acidic group include sulfo group, sodium sulfonato group, calcium sulfonato group, strontium sulfonato group, barium sulfonato group, aluminum sulfonato group, 4-(aluminum sulfonato)phenylcarbamoylmethyl group, dodecylammonio sulfonato group, octadecylammonio sulfonato group, trimethyloctadecylammonio sulfonato group, dimethyldidecylammonio sulfonato group, carboxyl group, 2-ammonium carboxylate-5-nitrobenzamidemethyl group and 4-carboxyphenylaminocarbonyl group. The benzoisoindole derivative contained in the pigment composition of the invention has high crystal growth preventive effect and particle stabilizing effect for the pigment, and the novel benzoisoindole derivative having the basic group or acidic group also has a high viscosity lowering effect. The proportion of the benzoisoindole derivative to the pigment derivative may be changed in a wide range depending on the purpose of use, and the proportion is preferably in the range of 100:0 to 30:70 in weight ratio in order to obtain a higher viscosity lowering effect.

The total amount of the benzoisoindole derivative, which is added during the pulverizing process and/or after pulverizing, and the pigment derivative, contained in the pigment composition of the invention is preferably from 0.1 to 35 parts by weight, more preferably from 1 to 30 parts by weight, relative to 100 parts by weight of the pigment. The effect of the pigment dispersant added is hardly obtained when the total amount of the benzoisoindole derivative of the invention and the pigment derivative is less than 0.1 part by weight. When the total amount is larger than 35 parts by weight, on the other hand, an effect corresponding to the amount of addition is not obtained while the difference in properties between the pigment composition obtained and individual pigments may become so large that the pigment composition may cause problems of the practical quality when used in inks and paints.

Next, the colored composition of the invention will be described.

The colored composition of the invention contains the pigment composition of the invention and a pigment carrier.

The pigment carrier is composed of a resin, a resin precursor or a mixture of the resin and precursor. When a color filter is produced by using the colored composition of the invention, it is preferable to use, as the resin, a transparent resin having a light transmissivity of 80% or more, particularly 95% or more, in an entire wavelength region of the visible light in the range of 400 to 700 nm.

Examples of the resin include a thermoplastic resin, thermosetting resin and photosensitive resin. Examples of the resin precursor include monomers or oligomers that form the resin upon curing by irradiation of radiation. These carriers may be used singly or in combination.

The pigment carrier may be used in an amount of 30 to 700 parts by weight, preferably 60 to 450 parts by weight, relative to 100 parts by weight of the pigment composition in the colored composition. When a mixture of the resin and resin precursor is used as the pigment carrier, the resin may be used in an amount of 20 to 400 parts by weight, preferably 50 to 250 parts by weight, relative to 100 parts by weight of the pigment composition in the colored composition. The resin precursor may be used in an amount of 10 to 300 parts by weight, preferably 10 to 200 parts by weight, relative to 100 parts by weight of the pigment composition in the colored composition.

Examples of the thermosetting resins include, for example, a butyral resin, a styrene-maleic acid copolymer, a chlorinated polyethylene, a chlorinated polypropylene, a polyvinyl chloride, a vinyl chloride-vinyl acetate copolymer, a polyvinyl acetate, a polyurethane resin, a polyester resin, an acrylic resin, an alkyd resin, a polystyrene resin, a polyamide resin, a rubber resin, a cyclized rubber resin, a cellulose, a polyethylene, a polybutadiene, and a polyimide resin.

Examples of the thermosetting resins include an epoxy resin, a benzoguanamine resin, a rosin-modified maleic acid resin, rosin-modified fumaric acid resin, a melamine resin, a urea resin, and a phenolic resin.

Examples of the photosensitive resin include a resin which is produced by introducing a photocrosslinkable group such as a (meth)acrylic compound or cinnamic acid into a linear polymer having a reactive substituent such as a hydroxyl group, a carboxyl group or an amino group, through an isocyanato group, an aldehydo group or an epoxy group. In addition, a polymer obtained by partial esterification of a linear polymer containing an acid anhydride, such as a styrene-maleic anhydride copolymer or an α-olefin-maleic anhydride copolymer, with a hydroxyl group-containing (meth)acrylic compound such as hydroxyalkyl (meth)acrylate can also be used.

Examples of the monomers and oligomers, which are the precursors of the resin, include, for example, various acrylic and methacrylic acid esters such as 2-hydroxyethyl (meth)acrylate, 2-hydroxypropyl (meth)acrylate, cyclohexyl (meth)acrylate, a polyethylene glycol di(meth)acrylate, pentaerythritol tri(meth)acrylate, dipentaerythritol hexa(meth)acrylate, tricyclodecanyl (meth)acrylate, hexa(meth)acrylate of caprolactone adduct of dipentaerythritol hexa(meth)acrylate, a melamine (meth)acrylate, and an epoxy (meth)acrylate prepolymer; (meth)acryl acid; styrene; vinyl acetate; hydroxylethyl vinyl ether; ethyleneglycol divinyl ether; pentaerythritol trivinyl ether; (meth)acrylamide; N-hydroxymethyl (meth)acrylamide; styrene; vinyl acetate; and acrylonitrile.

The colored composition according to the invention may appropriately contain a dispersion aid such as a surfactant or a resin type pigment dispersant for improving dispersibility of the pigment in the pigment carrier. The dispersion aid may be used in an amount of 0.1 to 40 parts by weight, preferably 0.1 to 30 parts by weight, relative to 100 parts by weight of the pigment composition in the colored composition.

Examples of the surfactant include, for example, anionic surfactants such as polyoxyethylene alkylether sulfate salts, sodium dodecylbenzenesulfonate, alkali salts of styrene-acryl acid copolymers, sodium alkylnaphthalenesulfonate, sodium alkyldiphenyletherdisulfonates, laurylsulfuric acid monoethanolamine, laurylsulfuric acid triethanolamine, ammonium laurylsulfate, stearic acid monoethanolamine, sodium stearate, sodium laurylsulfate, monoethanolamine salts of styrene-acryl acid copolymers, and polyoxyethylene alkylether phosphoric acid esters; nonionic surfactants such as polyoxyethylene oleylethers, polyoxyethylene laurylethers, polyoxyethylene nonylphenylethers, polyoxyethylene alkylether phosphoric acid esters, polyoxyethylene sorbitan monostearates, and polyethyleneglycol monolaurate; cationic surfactants such as quaternary alkyl ammonium salts and ethylene oxide adducts thereof; alkyl betaines such as alkyldimethylaminoacetic acid betaines; amphoteric surfactants such as alkylimidazolines; and other surfactants such as fluorosurfactants and silicone surfactants. These surfactants may be used alone or in combination.

The resin type pigment dispersant has a pigment-affinity portion having a property of adsorbing on a pigment, and a portion compatible with the pigment carrier, and functions to stabilize the dispersion of a pigment in the pigment carrier by being adsorbed on the pigment. Examples of the resin type pigment dispersant include linear or comb polyester, acrylic and urethane resins. Of these, linear resins having a basic group, acidic group or an aromatic group on their main chain or terminal, or comb resin having a basic group, acidic group or an aromatic group on their main chain or side chain are preferred.

Examples of commercially available resin type pigment dispersants include POLYFLOW No. 75, No. 90 and No. 95 (trade names, manufactured by Kyoei-Sha Oil and Fat Chemical Industry Co.), MEGAFAC F171, F172 and F173 (trade names, manufactured by Dainippon Ink & Chemicals, Inc.), FLORAD Fc430 and Fc431 (trade names, manufactured by Sumitomo 3M Co.), SOL-SPERSE dispersants such as SOL-SPERSE 13240, 20000, 24000, 26000 and 28000 (trade names, manufactured by Avecia Co.), DISPER BIG dispersants such as DISPER BIG 111, 160, 161, 162, 163, 164, 170, 182, 2000 and 2001 (trade names, manufactured by Big Chemie Co.), AJISUPER dispersants such as AJISUPER PB711, PB411, PB111, PB814, PB821 and PB822 (trade names, manufactured by Ajinomoto Fine Techno Co.), and EFKA dispersants such as EFKA 46 and 47 (trade names, manufactured by Efka Chemicals Co.).

The colored composition of the present invention contains a photopolymerization initiator when the composition is cured by light irradiation such as UV irradiation.

Examples of the photopolymerization initiator include, for example, acetophenone photopolymerization initiators such as 4-phenoxy-dichloroacetophenone, 4-t-butyl-dichloroacetophenone, diethoxyacetophenone, 1-(4-isopropylphenyl)-2-hydroxy-2-methylpropan-1-one, 1-hydroxycyclohexylphenylketone, and 2-benzyl-2-dimethylamino-1-(4-morpholinophenyl)-butan-1-one; benzoin photopolymerization initiators such as benzoin, benzoin methylether, benzoin ethylether, benzoin isopropylether, and benzyldimethyl ketal; benzophenone photopolymerization initiators such as benzophenone, benzoylbenzoic acid, methyl benzoylbenzoate, 4-phenylbenzophenone, hydroxybenzophenone, acrylated benzophenones, and 4-benzoyl-4'-methyl diphenylsulfide; thioxanthone photopolymerization initiators such as thioxanthone, 2-chlorothioxanthone, 2-methylthioxanthone, isopropylthioxanthone, and 2,4-diisopropylthioxanthone; triazine photopolymerization initiators such as 2,4,6-trichloro-s-triazine, 2-phenyl-4,6-bis(trichloromethyl)-s-triazine, 2-(p-methoxyphenyl)-4,6-bis(trichloromethyl)-s-triazine, 2-(p-tolyl)-4,6-bis(trichloromethyl)-s-triazine, 2-piperonyl-4,6-bis(trichloromethyl)-s-triazine, 2,4-bis(trichloromethyl)-6-styryl-s-triazine, 2-(naphto-1-yl)-4,6-bis(trichloromethyl)-s-triazine, 2-(4-methoxy-naphto-1-yl)-4,6-bis(trichloromethyl)-s-triazine, 2,4-trichloromethyl-(piperonyl)-6-triazine, and 2,4-trichloromethyl(4'-methoxy styryl)-6-triazine; borate photopolymerization initiators; carbazole photopolymerization initiators; and imidazole photopolymerization initiators. The photopolymerization initiator may be used in an amount of 5 to 200 parts by weight, preferably 10 to 150 parts by weight, relative to 100 parts by weigh of the pigment composition in the colored composition.

The photopolymerization initiators can be used alone or in combination, but may be used together with a sensitizer, such as α-acyloxime ester, acylphosphine oxide, methylphenyl glyoxylate, benzil, 9,10-phenanthrenequinone, camphorquinone, ethylanthraquinone, 4,4'-diethylisophthalophenone, 3,3',4,4'-tetra(t-butylperoxycarbonyl)benzophenone or 4,4'-diethylaminobenzophenone. The sensitizer may be used in an amount of 0.1 to 60 parts by weight, relative to 100 parts by weight of the photopolymerization initiator used.

The colored composition according to the invention may contain a solvent in order to sufficiently disperse the pigment material into the pigment carrier and to make it easier to obtain a uniform coating. Examples of the solvent include, for example, cyclohexanone, propyleneglycol diacetate, propyleneglycol monomethyl ether, diethyleneglycol diethyl ether, methyl isobutyl keton, n-butyl alcohol, dipropyleneglycol monomethyl ether, tripropyleneglycol monomethyl ether, propyleneglycol monopropyl ether, dipropyleneglycol monopropyl ether, propyleneglycol monobutyl ether, dipropyleneglycol monobutyl ether, tripropyleneglycol monobutyl ether, propyleneglycol phenyl ether, dipropyleneglycol monomethyl ether acetate, 1,3-butyleneglycol diacetate, ethyleneglycol monobutyl ether, ethyleneglycol monobutyl ether acetate, diethyleneglycol monobutyl ether, diethyleneglycol monobutyl ether acetate, 3-methoxybutanol, 1,3-butyleneglycol, triacetin, 3,3,5-trimethyl-2-cyclohexen-1-one, ethyleneglycol monoethyl ether, γ-butyrolactone, isoamyl acetate, ethyl 3-ethoxypropionate, propylenecglycol monomethyl ether acetate, toluene, o-xylene, m-xylene, p-xylene, methyl 3-methoxypropionate, butyl acetate, isobutyl acetate, and propyl acetate. These solvents may be used alone or in combination.

The amount of the solvents used may be 800 to 4,000 parts by weight, preferably 1000 to 2500 parts by weight, relative to 100 parts by weight of the pigment composition in the colored composition.

The colored composition of the present invention can contain a storage stabilizer in order to stabilize the viscosity with time of the composition, and can contain an adhesion enhancer such as a silane coupling agent, in order to enhance the adhesion with the substrate.

Additives such as a heat polymerization inhibitor, plasticizer, surface protecting agent, lubricant, coating aid, adhesivity improving agent, and coating improving agent or development improving agent may be optionally added to the colored composition of the invention in the range not impairing the effect of the invention.

The colored composition of the invention may be produced by finely dispersing the pigment composition of the invention in the pigment carrier and organic solvent, optionally with the dispersion aid and photopolymerization initiator, using various dispersion means such as a three-roll mill, two-roll mill, horizontal sand mill, vertical sand mill, annular beads mill, kneader and attritor. A colored composition containing two or more kinds of pigments may be produced by mixing respective pigments that have been finely dispersed in the pigment carrier and organic solvent using the benzoisoindole derivative in separate runs.

While the colored composition of the invention may be dispersed after mixing all the components constituting the colored composition, it is preferable that the pigment and benzoisoindole derivative are dispersed using a part of the resin and/or organic solvent at first, and then the remaining components are added and dispersed.

Pre-dispersion using a kneading-mixing machine such as a kneader and three-roll mill, solid state dispersion with a two-roll mill, or treatment of the pigment with the benzoisoindole and pigment derivatives may be simultaneously or independently applied before dispersion with the horizontal sand mill, vertical sand mill, annular beads mill or attritor. A post-treatment such as aging by storing several hours to 1 week at 30 to 80°C in a warm state after dispersion with the beads mill, and a post-treatment using an ultrasonic dispersion machine or a collision type beads-less dispersion machine are effective for stabilizing the colored composition. Various dispersion machines and mixing machines such as a microfluidizer, high speed mixer, homomixer, ball mill, roll mill, stone mill and ultrasonic dispersion machine may be also used for producing the colored composition of the invention.

When the colored composition of the invention is used for producing a color filter, coarse grains with a size of 5 µm or more, preferably with a size of 1 µm or more, and more preferably with a size of 0.5 µm or more, and mingled dusts are preferably removed by means of centrifugation or filtration with a sintered filter or a membrane filter.

The colored composition of the invention may be prepared as a gravure offset printing ink, waterless offset printing ink, silk screen printing ink, or solvent-developing or alkali-developing colored resist material.

The solvent-developing or alkali-developing colored resist material is prepared by dispersing the pigment composition of the invention in a composition containing a thermoplastic resin, heat curable resin or photosensitive resin as a carrier of the pigment, a monomer, a photopolymerization initiator and a solvent.

The colored composition of the invention provides a coloring material that is excellent in dispersion effect and time-dependent stability with high staining power for coloring a wide range of printing inks and paints, and ink-jet inks as well as for coloring plastics.

The colored composition may be produced by the above-mentioned method without using any monomers or photopolymerization initiators, but instead using, in addition to the above resin and solvent, a resin such as a petroleum resin, casein, shellac, drying oil or synthetic drying oil, and a solvent such as ethyl cellosolve acetate, butyl cellosolve acetate, ethyl benzene, ethyl acetate, methanol, ethanol, isopropyl alcohol, butanol, ethyleneglycol, glycerin, dimethylformamide, SOLVESSO 100 (Exxon Chemical Co.), SWAZOL 1000 or petroleum solvent, optionally with a surfactant and/or a resin type pigment dispersant.

The invention will be described hereinafter with reference to production examples of the benzoisoindole derivative and examples using the same. In the examples, "parts" refers to "parts by weight", and "%" refers to "% by weight".

### Production Example 1: Synthesis of Compound (1)

1000 mL of carbon tetrachloride was added to 50g of Compound (I) shown below under cooling with ice. After dropwise addition of 200g of chlorosulfonic acid over 2 hours, the mixture was stirred for 1 hour at 55°C. 75g of thionyl chloride was added dropwise to the mixture followed by stirring for additional 2 hours. After cooled to room temperature, the reaction mixture was poured onto 500g of ice, followed by separating the organic layer. By re-precipitation, 40g of Compound (1) was obtained. The carbon tetrachloride solution of the reaction mixture after cooling to room temperature may be directly used for the next reaction, unless it causes any problems. The product was identified by ¹H-NMR, time-of-flight mass spectroscopy (TOFMS) and elementary analysis.
¹H-NMR (270 MHz, CDCl₃): δ 6.21 (2H, br-s, NH₂), 7.23-7.88 (8H, m, Ar-H), 8.28(1H, br-s, NH).
TOFMS; Calculated: 386.01; Found: 386.22.
Elemental Analysis (C₁₈H₁₁ClN₂O₄S); Calculated: C, 55.89; H, 2.87; N, 7.24; Found: C, 55.78; H, 2.73; N, 7.44.

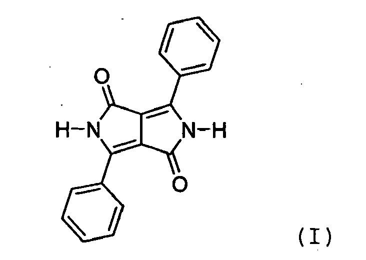

### Production Example 2: Synthesis of Compound (2)

5.21g of Compound (1) was stirred for 5 hours at 80°C in a mixed solvent of 50 mL of THF and 200 mL of distilled water. After cooling the reaction solution to room temperature, and the solution was filtered to give 4.33g of Compound (2). The product was identified by ¹H-NMR, TOFMS and elementary analysis.
¹H-NMR (270 MHz, DMSO-d₆): δ 5.83 (2H, br-s, NH₂), 7.38-7.94 (8H, m, Ar-H), 8.36 (1H, br-s, NH).
TOFMS; Calculated: 368.05; Found: 368.22.
Elemental Analysis (C₁₈H₁₂N₂O₅S); Calculated: C, 58.69; H, 3.28; N, 7.60; Found: C, 58.72; H, 3.33; N, 7.71.

### Production Example 3: Synthesis of Compound (3)

10.4g of Compound (2) and 200 mL of dilute sulfuric acid were heated at 150°C for 3 hours in an autoclave. The reaction solution was poured onto 500g of ice, which was filtered to give 7.78g of Compound (3). The product was identified by ¹H-NMR, TOFMS, elementary analysis and single crystal X-ray structure analysis.
¹H-NMR (270 MHz, CDCl₃): δ 4.84 (2H, br-s, NH₂), 7.38-7.72 (9H, m, Ar-H), 8.57 (1H, br-s, NH).
TOFMS; Calculated: 288.09; Found: 288.22
Elemental Analysis (C₁₈H₁₂N₂O₂); Caluculated: C, 74.99; H, 4.20; N, 9.72; Found: C, 75.03; H, 4.31; N, 9.85.

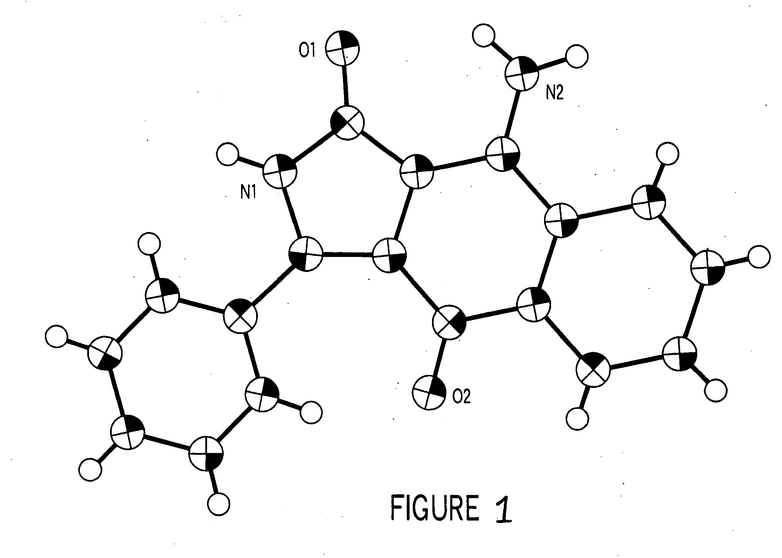

FIG. 1 shows an ORTEP (Oak Ridge Thermal Ellipsoid Plot) figure obtained from the single crystal X-ray structure analysis of Compound (3).

### Production Example 4: Synthesis of Compound (35)

11.2g of Compound (3) was dissolved in 40 mL of dimethylsulfoxide, to which 17.1 mL of triethylamine was added, and the solution was stirred. To this solution, a solution of 6.7 mL of bis(2-bromoethyl)ether in 30 mL of dimethylsulfoxide was added dropwise over 15 minutes at room temperature. After stirring the mixture for 72 hours at room temperature, extraction with ethyl acetate was carried out to give 10.5g of Compound (35). The product was identified by ¹H-NMR, ¹³C-NMR, TOFMS and elementary analysis.
¹H-NMR (270 MHz, CDCl₃): δ 3.19 (4H, t, J = 7.1 Hz, NCH₂), 3.74 (4H, t, J = 7.1 Hz, OCH₂), 7.32-7.83 (9H, m, Ar-H), 8.15 (1H, br-s, NH).
¹³C-NMR (67.5 MHz, CDCl₃): δ 49.2, 68.3, 101.9, 111.9, 123.6, 126.9, 128.4, 129.5, 128.3, 129.6, 128.7, 133.6, 134.6, 134.8, 139.2, 159.2, 169.6, 178.6.
TOFMS; Calculated: 358.13; Found: 358.32
Elemental Analysis (C₂₂H₁₈N₂O₃); Calculated: C, 73.73; H, 5.06; N, 7.82; Found: C, 73.79; H, 5.11; N, 7.76.

### Production Example 5: Synthesis of Compound (49)

28.2g of Compound (3) and 14.8g of trimethyl phosphate were mixed and stirred at 150°C for 45 minutes, followed by cooling the reaction solution to room temperature. To this solution, an aqueous sodium hydroxide solution (prepared by dissolving 20g of sodium hydroxide in 100 mL of distilled water) was added, and the mixed solution was vigorously stirred at room temperature. The reaction solution was cooled so that the temperature does not exceed 50°C since the heat of reaction is slowly emitted in the solution. After stirred for 3 hours, the reaction mixture was extracted with chloroform, and subjected to column chromatography to give 12.9g of Compound (49). The product was identified by ¹H-NMR, TOFMS and elementary analysis.
¹H-NMR (270 MHz, CDCl₃): δ 3.25 (6H, s, CH₃), 7.46-7.77 (9H, m, Ar-H), 8.33 (1H, br-s, NH).
TOFMS; Calculated: 316.12; Found: 316.27
Elemental Analysis (C₂₀H₁₆N₂O₂); Calculated: C, 75.93; H, 5.10; N, 8.86; Found: C, 76.01; H, 5.13; N, 8.68.

### Production Example 6: Synthesis of Compound (7)

Ice, 250g, was added to 11.5g of Compound (3), and 50 mL of conc. hydrochloric acid was slowly added. Then, 20 mL of an aqueous solution of 3.3g of sodium nitrite was added dropwise over 30 minutes while the temperature of the reaction solution was maintained at 5°C or less. After stirring the solution for 3 hours under cooling with ice, the precipitates were quickly filtered at the low temperature to obtain Compound (7). The filtered sample at the low temperature was promptly used for the next reaction in the synthetic reaction using Compound (7). While Compound (7) was not identified in this step since it is an unstable substance, production of Compound (7) is suggested since Compound (22) synthesized in Production Example 7 could be identified.

### Production Example 7: Synthesis of Compound (22)

Compound (7) prepared from 48g of Compound (3) according to Production Example 6 was dissolved in 30 mL of toluene, to which 12.1g of sodium nitrite was added, and the solution was stirred for 1 hour under cooling with ice. The temperature was gradually increased, and stirring was continued for additional 5 hours at room temperature. The reaction mixture was precipitated with methanol to give 30g of Compound (22). The product was identified by ¹H-NMR, ¹³C-NMR, TOFMS and elementary analysis.
¹H-NMR (270 MHz, CDCl₃): δ 7.36-7.82 (9H, m, Ar-H), 8.44 (1H, br-s, NH).
¹³C-NMR (67.5 MHz, CDCl₃): δ 111.7, 123.0, 127.3, 129.1, 129.4, 128.8, 128.5, 129.2, 132.8, 134.9, 135.7, 139.7, 140.3, 154.3, 169.3, 178.3.
TOFMS; Calculated: 318.06; Found: 318.07. Elemental Analysis (C₁₈H₁₀N₂O₄); Calculated: C, 67.92; H, 3.17; N, 8.80; Found: C, 67.90; H, 3.31; N, 8.79.

### Production Example 8: Synthesis of Compound (57)

5 mL of an aqueous solution of 1.73g of hydrazine monohydrate was added dropwise to a THF solution of Compound (1) prepared from 2.8g of Compound (I) according to Production Example 1 under cooling with ice, and the mixture was vigorously stirred for 4 hours. The reaction mixture was subjected to extraction with ethyl acetate, and precipitation was carried out to give 2.0g of Compound (57). The product was identified by ¹H-NMR, TOFMS and elementary analysis.
¹H-NMR (270 MHz, CDCl₃): δ 7.29-7.72 (9H, m, Ar-H), 8.21 (1H, br-s, NH).
TOFMS; Calculated: 298.07; Found: 298.63.
Elemental Analysis (C₁₉H₁₀N₂O₂); Calculated: C, 76.50; H, 3.38; N, 9.39; Found: C, 76.73; H, 3.44; N, 9.31.

### Production Example 9: Synthesis of Compound (31)

14.5g of an aqueous aluminum sulfate solution (8% as alumina) was added dropwise to 8.30g of Compound (2) in a mixed solvent of 50 mL of methanol and 200 mL of distilled water over 20 minutes, and the mixture was stirred at 80°C for 2 hours. After cooling the reaction solution to room temperature, the reaction solution was filtered to give 6.06g of Compound (31). The product was identified by ¹H-NMR, TOFMS and elementary analysis.
¹H-NMR (270 MHz, CDCl₃): δ 4.33 (6H, br-s, NH₂), 7.36-7.78 (24H, m, Ar-H).
TOFMS; Calculated: 1128.10; Found: 1128.55.
Elemental Analysis (C₅₄H₃₃AlN₆0₁₅S₃); Calculated: C, 57.44; H, 2.95; N, 7.44; Found: C, 57.61; H, 3.11; N, 7.66.

### Production Example 10: Synthesis of Compound (33)

Compound (33) was obtained by the same procedures as in Production Example 9, except that a solution of trimethyldodecylammonium chloride in a mixed solvent of IPA and water was used in place of the aqueous solution of aluminum sulfate. The product was identified by ¹H-NMR, TOFMS and elementary analysis.

¹H-NMR (270 MHz, DMSO-d₆): δ 0.82 (3H, t, J = 7.4 Hz, CH₂CH₃), 1.25-1.69 (20H, m, NCH₂(CH₂)₁₀CH₃), 3.14 (2H, t, J = 7.1 Hz, NCH₂), 3.31 (9H, s, NCH₃), 4.62 (2H, br-s, NH₂), 7.46-7.91 (8H, m, Ar-H), 9.10 (1H, br-s, NH).
TOFMS; Calculated: 595.31; Found: 595.47.
Elemental Analysis (C₃₃H₄₅N₃O₅S) ; Calculated: C, 66.53; H, 7.61; N, 7.05; Found: C, 66.32; H, 7.55; N, 7.28.

### Production Example 11: Synthesis of Compound (60)

7.20g of Compound (3) was added to 70 mL of conc. sulfuric acid, and 4.54g of n-hydroxymethyl phthalimide was slowly added while the temperature was kept at 10°C or less. The reaction mixture was stirred for 1 hour, heated to 60°C and was allowed to react for 2 hours. The reaction mixture was poured into 500 mL of water, filtered and washed with water to give 7.48g of Compound (60). The product was identified by ¹H-NMR, TOFMS and elementary analysis.
¹H-NMR (270 MHz, CDCl₃): δ 4.51 (2H, br-s, NH₂), 4.82 (2H, s, CH₂), 7.11-7.89 (12H, m, Ar-H), 8.22 (1H, br-s, NH).
TOFMS; Calculated: 447.12; Found: 447.55.
Elemental Analysis (C₂₇H₁₇N₃O₄); Calculated: C, 72.48; H, 3.83; N, 9.39; Found: C, 72.52; H, 3.61; N, 9.14.

### Production Example 12: Synthesis of Compound (25)

4.5g of 2-aminoethanesulfonic acid was dissolved in 50 mL of water, and Compound (1) prepared from 8.7g of Compound (I) according to Production Example 1 was slowly added. Then, 1.8g of sodium carbonate was added, and the mixture was heated to 65°C, then stirred for 1 hour, and was allowed to precipitate, giving 10.9 f of Compound (25). The product was identified by ¹H-NMR, TOFMS and elementary analysis.
¹H-NMR (270 MHz, DMSO-d₆): δ 3.32 (2H, t, J = 6.9 Hz, CH₂), 3.91 (2H, t, J = 6.9 Hz, CH₂), 7.38-7.72 (8H, m, Ar-H).
TOFMS; Calculated: 475.05; Found: 475.66.
Elemental Analysis (C₂₀H₁₇N₃O₇S₂); Calculated: C, 50.52; H, 3.60; N, 8.84; Found: C, 50.51; H, 3.51; N, 8.68.

### Production Example 13: Synthesis of Compound (68)

10.1g of Compound (3) was dissolved in a mixture of 35g of conc. sulfonic acid and 70g of chlorosulfonic acid. 15.8g of paraformaldehyde was added, and the mixture was stirred at 45°C for 1 hour. Then, 8.9g of diethylamine was added dropwise over 15 minutes, followed by stirring for additional 2 hours. 8.6g of Compound (68) was obtained by precipitation. The product was identified by ¹H-NMR, ¹³C-NMR, TOFMS and elementary analysis.
¹H-NMR (270 MHz, CDCl₃): δ 1.06 (6H, t, J = 7.2 Hz, CH₃), 2.58 (4H, q, J = 7.2 Hz, CH₃CH₂), 3.59 (2H, s, CH₂), 4.48 (2H, br-s, NH₂), 7.08-7.52 (8H, m, Ar-H), 8.21 (1H, br-s, NH).
¹³C-NMR (67.5 MHz, CDCl₃): 13.3, 46.6, 62.5, 105.9, 112.2, 123.5, 126.6, 129.4, 129.2, 129.6, 129.5, 132.9, 134.4, 135.3, 137.8, 139.5, 156.1, 168.4, 178.5.
TOFMS; Calculated: 373.18; Found: 373.54
Elemental Analysis (C₂₃H₂₃N₃O₂); Calculated: C, 73.97; H, 6.21; N, 11.25; Found: C, 74.07; H, 6.05; N, 11.39.

### Production Example 14: Synthesis of Compound (36)

A THF (8 mL) solution of 3.0g of diethylamine was added dropwise to a carbon tetrachloride solution of Compound (1) prepared from 5.8g of Compound (I) according to Production Example 1, over 30 minutes under cooling with ice. After heating the reaction mixture at 40°C, the mixture was stirred for 1 hour followed by re-precipitation to give 2.0g of Compound (36). The product was identified by ¹H-NMR, TOFMS and elementary analysis.
¹H-NMR (270 MHz, CDCl₃): δ 1.01 (6H, t, J = 7.0 Hz, CH₃), 3.31 (4H, q, J = 7.0 Hz, CH₃CH₂), 4.58 (2H, br-s, NH₂), 7.48-7.72 (8H, m, Ar-H), 8.05 (1H, br-s, NH).
TOFMS; Calculated: 423.13; Found: 423.62.
Elemental Analysis (C₂₂H₂₁N₃O₄S); Calculated: C, 62.40; H, 5.00; N, 9.92; Found: C, 62.31; H, 5.12; N, 9.88.

### Production Examples 15 to 28

Fourteen compounds shown in Table 2 were obtained by the same procedures as in Production Example 14, except that amines shown in Table 2 were used in place of diethylamine. The product was identified by ¹H-NMR and MALDI-TOFMS. In Table 2, Me denotes methyl group, Et denotes ethyl group, Pr denotes normal propyl group, i-Pr denotes 2-propyl group and Bu denotes butyl group.

**Table 2**

| | Amine used | Synthesized Compound | | Amine used | Synthesized Compound |
|---|---|---|---|---|---|
| Prod. Ex. 15 | 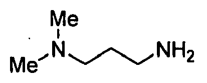 | Compound (79) | Prod. Ex. 16 | 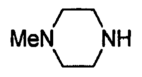 | Compound (84) |
| Prod. Ex. 17 | 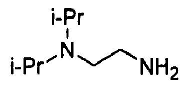 | Compound (87) | Prod. Ex. 18 | 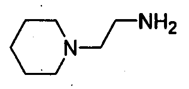 | Compound (91) |
| Prod. Ex. 19 | 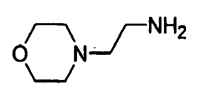 | Compound (92) | Prod. Ex. 20 | 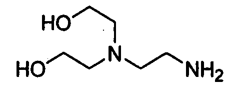 | Compound (93) |
| Prod. Ex. 21 | 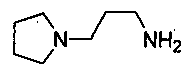 | Compound (95) | Prod. Ex. 22 | 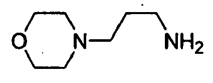 | Compound (97) |
| Prod. Ex. 23 | 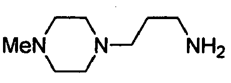 | Compound (98) | Prod. Ex. 24 | 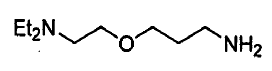 | Compound (102) |
| Prod. Ex. 25 | 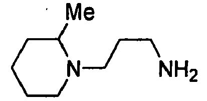 | Compound (104) | Prod. Ex. 25 | 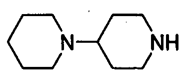 | Compound (105) |
| Prod. Ex. 27 | 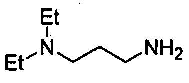 | Compound (109) | Prod. Ex. 28 | 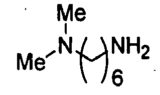 | Compound (111) |

### Example 1

47.5 parts of diketopyrrolopyrrole red pigment (C.I. Pigment Red 254, manufactured by Ciba Specialtiy Chemicals Co., trade name: IRGAPHOR RED 2030), 2.5 parts of Compound (3) and 2,000 parts of steel beads (8 mm in diameter) were charged in a dry attritor with a tank volume of 0.8L, and pulverized at a rotation speed of 300 rpm at 60°C for 4 hours to afford a pigment composition.

### Example 2

45 parts of diketopyrrolopyrrole red pigment as in Examples 1, 5 parts of Compound (33) and 2,000 parts of steel beads (8 mm in diameter) were charged in a dry attritor with a tank volume of 0.8L, and pulverized at a rotation speed of 300 rpm at 60°C for 4 hours to afford a pigment composition.

### Example 3

96 parts of diketopyrrolopyrrole red pigment as in Examples 1, 4 parts of Compound (60), 450 parts of sodium chloride and 100 parts of diethyleneglycol were charged in a double-arm kneader with a volume of 1000 parts by volume, and the mixture was pulverized by a solvent salt milling method for 6 hours at 80°C, while keeping the mixture as a dense mass (dough) state. The mixture was discharged in water at 70°C, stirred for 1 hour at 70°C, and was filtered and washed with water to remove sodium chloride and diethyleneglycol. The resultant material was then dried at 80°C and pulverized to afford a pigment composition.

### Example 4

95 parts of diketopyrrolopyrrole red pigment as in Examples 1, 5 parts of Compound (31), 450 parts of sodium chloride and 100 parts of diethyleneglycol were charged in a double-arm kneader with a volume of 1000 parts by volume. The mixture was pulverized by the solvent salt milling method as in Example 3 to afford a pigment composition.

### Example 5

A pigment composition was obtained by the same procedures as in Example 4, except that Compound (49) was used in place of Compound (31).

### Example 6

92 parts of diketopyrrolopyrrole red pigment as in Examples 1, 8 parts of Compound (79), 450 parts of sodium chloride and 100 parts of diethyleneglycol were charged in a double-arm kneader with a volume of 1000 parts by volume. A pigment composition was obtained by pulverizing the mixture by the solvent salt milling method as in Example 3.

### Example 7

A pigment composition was obtained by the same procedures as in Example 6, except that Compound (95) was used in place of Compound (79).

### Example 8

A pigment composition was obtained by the same procedures as in Example 7, except that 84 parts of diketopyrrolopyrrole red pigment as in Example 1 and 16 parts of Compound (95) were used.

### Example 9

A pigment composition was obtained by the same procedures as in Example 4, except that Compound (22) was used in place of Compound (31).

### Example 10

A pigment composition was obtained by the same procedures as in Example 6, except that Compound (91) was used in place of Compound (79).

### Example 11

92 parts of diketopyrrolopyrrole red pigment as in Example 1, 4 parts of Compound (60), 4 parts of a diketopyrrolopyrrole derivative having a diethylaminopropylaminosulfonyl group (compound A), 450 parts of sodium chloride and 100 parts of diethyleneglycol were charged in a double-arm kneader with a volume of 1000 parts by volume, and a pigment composition was obtained by pulverizing the mixture by the solvent salt milling method as in Example 3.

### Example 12

A pigment composition was obtained by the same procedures as in Example 6, except that quinacridone red pigment (C.I. Pigment Red 122, manufactured by Clariant Co., trade name: Hostaperm pink E) was used in place of diketopyrrolopyrrole red pigment.

### Example 13

92 parts of quinacridone red pigment as in Example 12, 8 parts of Compound (98), 450 parts of sodium chloride and 100 parts of diethyleneglycol were charged in a double arm kneader with a volume of 1000 parts by volume, and a pigment composition was obtained by pulverizing the mixture by the solvent salt milling method as in Example 3.

### Example 14

A pigment composition was obtained by the same procedures as in Example 6, except that quinacridone red pigment (C.I. Pigment Violet 19, manufactured by Toyo Ink Manufacturing Co., trade name: LIONOGEN RED 5700) was used in place of diketopyrrolopyrrole red pigment.

### Example 15

90 parts of anthraquinone red pigment (C.I. Pigment Red 177, manufactured by Ciba Speciality Chemicals Co., trade name: CHROMOPHTAL RED A2B), 10 parts of Compound (25), 450 parts of sodium chloride and 100 parts of diethyleneglycol were charged in a double-arm kneader with a volume of 1000 parts by volume. A pigment composition was obtained by pulverizing the mixture by the solvent salt milling method as in Example 3, except that the time of solvent salt milling treatment was changed from 6 hours to 5 hours.

### Example 16

A pigment composition was obtained by the same procedures as in Example 15, except that Compound (68) was used in place of Compound (25).

### Comparative Example 1

50 parts of diketopyrrolopyrrole red pigment as in Example 1 and 2,000 parts of steel beads (8 mm in diameter) were charged in a dry attritor with a tank volume of 0.8L, and a pigment composition was obtained by the same procedures as in Example 1.

### Comparative Example 2

100 parts of diketopyrrolopyrrole red pigment as in Example 1, 450 parts of sodium chloride and 100 parts of diethyleneglycol were charged in a double-arm kneader with a volume of 1000 parts by volume, and a pigment composition was obtained by pulverizing the mixture by the solvent salt milling method as in Example 3.

### Comparative Example 3

A pigment composition was obtained by the same procedures as in Comparative Example 2, except that the time of solvent salt milling treatment was prolonged from 6 hours to 9 hours.

### Comparative Example 4

A pigment composition was obtained by the same procedures as in Comparative Example 2, except that quinacridone red pigment was used as in Example 12 in place of diketopyrrolopyrrole red pigment.

### Comparative Example 5

A pigment composition was obtained by the same procedures as in Comparative Example 2, except that anthraquinone red pigment was used as in Example 15 in place of diketopyrrolopyrrole red pigment.

The pigment compositions obtained in Examples 1 to 16 and Comparative Examples 1 to 5 were observed with an electron microscope, and average particle diameter and distribution range of the particle diameter were determined. The results are summarized in Table 3.

**Table 3**

| Ex. | Pigment | Benzoisoindole derivative | Amount of Benzo-isoindole deriv. (%) | Average particle diameter (nm) | Distribution range of particle diameter (nm) |
|---|---|---|---|---|---|
| 1 | C.I. Pig. Red 254 | Compound (3) | 5 | 45 | 25-80 |
| 2 | C.I. Pig. Red 254 | Compound (33) | 10 | 60 | 30-120 |
| 3 | C.I. Pig. Red 254 | Compound (60) | 4 | 29 | 25-35 |
| 4 | C.I. Pig. Red 254 | Compound (31) | 5 | 40 | 25-50 |
| 5 | C.I. Pig. Red 254 | Compound (49) | 5 | 35 | 25-50 |
| 6 | C.I. Pig. Red 254 | Compound (79) | 8 | 24 | 20-30 |
| 7 | C.I. Pig. Red 254 | Compound (95) | 8 | 38 | 20-50 |
| 8 | C.I. Pig. Red 254 | Compound (95) | 16 | 33 | 20-40 |
| 9 | C.I. Pig. Red 254 | Compound (22) | 5 | 34 | 25-50 |
| 10 | C.I. Pig. Red 254 | Compound (91) | 8 | 38 | 20-50 |
| 11 | C.I. Pig. Red 254 | Compound (60) | 4 | 25 | 20-30 |
| | | Compound A | 4 | | |
| 12 | C.I. Pig. Red 122 | Compound (79) | 8 | 26 | 20-35 |
| 13 | C.I. Pig. Red 122 | Compound (98) | 8 | 28 | 25-35 |
| 14 | C.I. Pig. Violet 19 | Compound (79) | 8 | 28 | 20-35 |
| 15 | C.I. Pig. Red 177 | Compound (25) | 10 | 32 | 25-45 |
| 16 | C.I. Pig. Red 177 | Compound (68) | 10 | 27 | 20-35 |

| Comp. Ex. | | | | | |
|---|---|---|---|---|---|
| 1 | C.I. Pig. Red 254 | - | - | 100 | 50-200 |
| 2 | C.I. Pig. Red 254 | - | - | 75 | 30-100 |
| 3 | C.I. Pig. Red 254 | - | - | 60 | 30-9.0 |
| 4 | C.I. Pig. Red 122 | - | - | 50 | 20-70 |
| 5 | C.I. Pig. Red 177 | - | - | 50 | 25-90 |

When the benzoisoindole derivative was used for pulverizing the pigment, fine particles with narrow particle size distribution could be readily obtained due to crystal growth preventive effect and particle stabilizing effect of the derivative, and the pigment composition was suitable for use in application fields where fine dispersion of the pigment particles is required.

### <Preparation of acrylic resin solution>

700g of cyclohexanone was added to a separable four-neck flask equipped with a thermometer, cooling pipe, nitrogen gas inlet tube, dripping tube, and stirrer, and was heated at 80°C. After purging the reaction vessel with nitrogen, a mixture of 133g of n-butyl methacrylate, 46g of 2-hydroxyethyl methacrylate, 43g of methacrylic acid, 74g of paracumylphenol ethyleneoxide-modified acrylate (trade name: ARONIX M110, manufactured by Toagosei Co.) and 4.0g of 2,2'-azobisisobutylonitrile was added dropwise from the dripping tube over 2 hours. The reaction was continued for additional 3 hours after completing the dropwise addition to give a solution of an acrylic resin with a weight average molecular weight of 26,000. After cooling the solution to room temperature, about 2g of the resin solution was dried by heating at 180°C for 20 minutes to measure the amount of nonvolatile fractions. Based on the measurement, cyclohexanone was added so that the proportion of the nonvolatile fraction of the resin solution was 20% to afford a desired acrylic resin solution.

### <Preparation of red/yellow mixed pigment 1>

Diketopyrrolopyrrole red pigment as in Example 1, anthraquinone red pigment as in Example 15 and azo yellow pigment (C.I. Pigment Yellow 150, manufactured by Lanxess Co., trade name: YELLOW PIGMENT E4GN-GT) were mixed at a weight ratio of 5:3:1.

### <Preparation of red/yellow mixed pigment 2>

Diketopyrrolopyrrole red pigment as in Example 1 and azo yellow pigment (C.I. Pigment Yellow 150, manufactured by Lanxess Co., trade name: YELLOW PIGMENT E4GN-GT) were mixed at a weight ratio of 4:1.

### Examples 17 to 31 and Comparative Examples 6 to 11

Each pigment composition or pigment, benzoisoindole derivative, acrylic resin solution shown above, cyclohexanone as a solvent, and optionally an additive (40% anone solution of AJISPER PB821, manufactured by Ajinomoto Fine Techno Co.) were weighed into a 140 mL screw-capped bottle in respective blending ratios shown in Table 4. Zirconia beads (150g) with a diameter of 1.25 mm were added to the bottle, and the mixture was dispersed with a paint conditioner for 8 hours to afford a colored composition.

### <Evaluation of colored composition>

Viscosity and thixotropy index value (TI value) of each colored composition at 25°C were measured with a Brookfield viscometer immediately after preparation and after preserving at 40°C for 1 week. The results are summarized in Table 4.

**Table 4**

| Ex. | Pigment composition or Pigment | | Derivative | | Additive | Acrilic resin sol. | Solv. | Initial Vis. (MPa·s) | | | Vis. after storage (MPa·s) | | |
|---|---|---|---|---|---|---|---|---|---|---|---|---|---|
| | Kind | Amount (g) | Compound No. | Amount (g) | Amount (g) | Amount (g) | Amount (g) | 6 rpm | 60 rpm | TI val. | 6 rpm | 60 rpm | TI val. |
| 17 | Ex. 1 | 11.40 | 109 | 0.60 | - | 29.7 | 42.3 | 76 | 62 | 1.23 | 387 | 199 | 1.84 |
| 18 | Ex. 3 | 10.80 | 109 | 1.20 | - | 29.7 | 42.0 | 78 | 66 | 1.18 | 116 | 95 | 1.22 |
| 19 | Ex. 4 | 10.80 | 31 | 1.20 | 2.40 | 24.9 | 40.6 | 108 | 90 | 1.20 | 148 | 115 | 1.29 |
| 20 | Ex. 6 | 11.00 | 79 | 1.00 | - | 29.7 | 41.3 | 61 | 53 | 1.15 | 80 | 73 | 1.10 |
| 21 | Ex. 7 | 12.00 | - | - | - | 29.7 | 43.8 | 172 | 119 | 1.48 | 1050 | 739 | 1.42 |
| 22 | Ex. 8 | 12.00 | - | - | - | 29.7 | 43.0 | 113 | 96 | 1.17 | 250 | 159 | 1.57 |
| 23 | Ex. 11 | 11.52 | 109 | 0.48 | - | 29.7 | 41.5 | 67 | 55 | 1.21 | 105 | 155 | 1.47 |
| 24 | Ex. 12 | 10.80 | 109 | 1.20 | - | 29.7 | 42.5 | 101 | 87 | 1.16 | 185 | 137 | 1.35 |
| 25 | Ex. 15 | 11.20 | 25 | 0.80 | 1.60 | 26.5 | 42.6 | 200 | 140 | 1.43 | 870 | 458 | 1.90 |
| 26 | Ex. 16 | 11.20 | 68 | 0.80 | - | 29.7 | 43.2 | 148 | 101 | 1.47 | 630 | 423 | 1.49 |
| 27 | C.I. Pig. Red 254 | 10.50 | 68 | 1.50 | - | 29.7 | 41.5 | 50 | 47 | 1.06 | 68 | 59 | 1.15 |
| 28 | C.I Pig.Red 122 | 10.50 | 68 | 1.50 | - | 29.7 | 42.0 | 70 | 62 | 1.13 | 94 | 79 | 1.19 |
| 29 | C.I Pig.Red 122 | 10.56 | 87 | 0.72 | - | 29.7 | 42.7 | 132 | 95 | 1.39 | 540 | 288 | 1.89 |
| | | | Compound B | 0.72 | | | | | | | | | |
| 30 | Red mixed pigment 1 | 10.50 | 79 | 1.50 - | | 29.7 | 42.7 | 91 | 73 | 1.25 | 130 | 93 | 1.40 |
| 31 | Red mixed pigment 2 | 10.50 | 109 | 1.50 | - | 29.7 | 42. | 2 63 | 55 | 1.15 | 90 | 72 | 1.25 |

| Comp. Ex. | | | | | | | | | | | | | |
|---|---|---|---|---|---|---|---|---|---|---|---|---|---|
| 6 | C.I.Pig.Red 254 | 12.00 | - | - | | 29.7 | 44.3 | 1290 | 871 | 1.48 | 12500 | 2590 | 4.83 |
| 7 | Comp. Ex. 3 | 12.00 | - | - | | 29.7 | 45.3 | 3740 | 1135 | 3.29 | 13100 | 2930 | 4.47 |
| 8 | C.I Pig. Red 122 | 12.00 | - | - | | 29.7 | 44.0 | 860 | 425 | 2.02 | 13800 | 2730 | 5.05 |
| 9 | Comp. Ex. 5 | 12.00 | - | - | | 29.7 | 45.3 | 4900 | 1960 | 3.52 | 27400 | 5000 | 5.48 |
| 10 | Red mixed pigment 1 | 12.00 | - | - | | 29.7 | 45.3 | 2300 | 1380 | 1.69 | 8090 | 1940 | 4.17 |
| 11 | Red mixed pigment 2 | 12.00 | - | - | | 29.7 | 45.3 | 1120 | 825 | 1.36 | 7900 | 2070 | 3.81 |

In Table 4, Compound B is a quinacridone derivative having a 4-aminophenylaminosulfonyl group.

The benzoisoindole derivatives obtained in Production Examples 14, 16, 19, 20, 22, 24, 25, 26 and 28 showed fining effects when used for pulverization of the pigment, and viscosity lowering effect and time-dependent stabilizing effect when used for dispersion of the pigment.

The colored compositions of the invention using the benzoisoindole derivative, in particular benzoisoindole derivative having a basic or acidic group, exhibited low viscosity, excellent fluidity and time dependent stability when used for pulverizing and/or dispersing the pigment, and gave a coating film excellent in glossiness.

The colored composition also showed excellent dispersibility, fluidity and time-dependent stability when used in a finely dispersed aqueous ink that contains 20% of the pigment composition together with ion-exchange water, glycerin, diethyleneglycol, monobutyl ether and styrene/maleic acid resin.

As hitherto described, pigment particles are pulverized in a high level due to high pigment crystal growth preventive effect and particle stabilizing effect of the novel benzoisoindole derivative in the pigment composition of the invention. These effects are exhibited when pulverizing or uniformizing the pigment particles. The pigment compositions containing novel benzoisoindole derivatives that have a basic group or an acidic group show excellent dispersibility in a wide variety of resins while inks and paints excellent in non-cohesivity, non-crystallinity, viscosity compatibility, glossiness of the coating film, good visibility and storage stability may be readily obtained. Accordingly, the pigment composition may be applicable to printing inks, various common paints for automobiles, woods and metals, back-coat paints for magnetic tapes, radiation-curable inks, ink-jet printer inks and color filter inks.

The pigment crystal growth preventive effect and particle stabilizing effect are particularly high in the systems containing red pigments represented by diketopyrrolopyrrole red pigments, quinacridone red pigments, thiazine-indigo red pigments and anthraquinone red pigments while the pigment composition affords high dispersibility, excellent viscosity compatibility and storage stability.

A pigment composition contains a specified benzoisoindole derivative, and a pigment. A colored composition contains such a pigment composition and a pigment carrier.

## Claims

1. A benzoisoindole derivative represented by Formula (1): 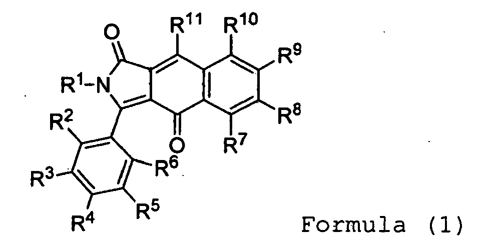 where:
R¹ denotes a hydrogen atom, an alkyl group that may have one or more substituents, or an acyl group that may have one or more substituents;
R², R³, R⁵, R⁶, R⁷, R⁸, R⁹ and R¹⁰ each independently denote a hydrogen atom, an alkyl group that may have one or more substituents, a phenyl group, a halogen atom, a cyano group, an alkoxy group that may have one or more substituents, or -NR¹²R¹³, where R¹² and R¹³ each independently denote a hydrogen atom or an alkyl group that may have one or more substituents, or R¹² and R¹³ may be bonded together to form a heterocyclic ring that may further contain a nitrogen, oxygen or sulfur atom;
R⁴ denotes a hydrogen atom, an alkyl group that may have one or more substituents, an acyl group that may have one or more substituents, or a substituent represented by Formula (2):
-Y-(Z)ₖ (2)
where Y denotes a direct bond, -O-, -S(O)ₘ- (where m denotes an integer of from 0 to 3), -CO-, -NR¹⁴-, - CONR¹⁴-, -SO₂NR¹⁴, -NR¹⁴CO-, NR¹⁴SO₂- (where R¹⁴ denotes a hydrogen atom, an alkyl group or a hydroxylalkyl group), a linear or branched alkylene having 1 to 12 carbon atoms, or a benzene residue or triazine residue that may be substituted with an alkyl group, an amino group, a nitro group, a hydroxyl group, an alkoxy group or a halogen atom, or a linking group formed of two or more of these linking groups; Z denotes a hydrogen atom, a halogen atom, an alkyl group that may have one or more substituents, an aryl group that may have one or more substituents, a phthalimidomethyl group that may have one or more substituents, -NR¹⁵R¹⁶, -SO₃-M/n or -COO·M/n, where R¹⁵ and R¹⁶ each independently denote a hydrogen atom, an alkyl group that may have one or more substituents, an alkenyl group that may have one or more substituents or a phenyl group that may have one or more substituents, or R¹⁵ and R¹⁶ may be bonded together to form a heterocyclic ring that may further contain a nitrogen, oxygen or sulfur atom and that may have one or more substituents; M denotes a hydrogen ion, a mono- to trivalent metal ion or an ammonium ion at least one hydrogen atom of which is substituted with an alkyl group; n denotes valence of M; and k denotes an integer of 1 or 2; and
R¹¹ denotes a hydrogen atom, a halogen atom, a nitro group, a cyano group, a hydroxyl group, an alkoxy group that may have one or more substituents, an aryloxy group that may have one or more substituents, -S(O)ₘR¹⁷, -NR¹⁸R¹⁹ or -N₂⁺X-, where m is an integer of from 0 to 3, R¹⁷ denotes a hydrogen atom, an alkyl group that may have one or more substituents, an aryl group that may have one or more substituents, or a halogen atom; R¹⁸ and R¹⁹ each independently denote a hydrogen atom or an alkyl group that may have one or more substituents, or R¹⁸ and R¹⁹ may be bonded together to form a heterocyclic ring that may further contain a nitrogen, oxygen or sulfur atom; and X denotes a halogen atom.

2. The benzoisoindole derivative according to claim 1, **characterized in that**:
R¹ denotes a hydrogen atom;
R², R³, R⁵, R⁶, R⁷, R⁸, R⁹ and R¹⁰ each independently denote a hydrogen atom, an alkyl group, a phenyl group, a halogen atom, a cyano group, an alkoxy group, or -NR¹²R¹³, where R¹² and R¹³ each independently denote a hydrogen atom or an alkyl group having 1 to 12 carbon atoms, or R¹² and R¹³ may be bonded together to form a heterocyclic ring that may further contain a nitrogen, oxygen or sulfur atom;
R⁴ denotes a hydrogen atom, an alkyl group that may have one or more substituents, an acyl group that may have one or more substituents, or a substituent represented by Formula (2) above; and
R¹¹ denotes a hydrogen atom, a halogen atom, a nitro group, a cyano group, a hydroxyl group, an alkoxy group, an aryloxy group, -S(O)ₘR¹⁷, -NR¹⁸R¹⁹ or -N₂⁺X⁻, where m is an integer of from 0 to 3, R¹⁷ denotes a hydrogen atom, an alkyl group, an aryl group, or a halogen atom; R¹⁸ and R¹⁹ each independently denote a hydrogen atom or an alkyl group having 1 to 12 carbon atoms, or R¹⁸ and R¹⁹ may be bonded together to form a heterocyclic ring, that may further contain a nitrogen, oxygen or sulfur atom; and X denotes a halogen atom.

3. The benzoisoindole derivative according to claim 2, **characterized in that** R¹¹ denotes a hydrogen atom or -NR¹⁸R¹⁹.

4. A pigment composition comprising a pigment and a benzoisoindole derivative represented by Formula (I): 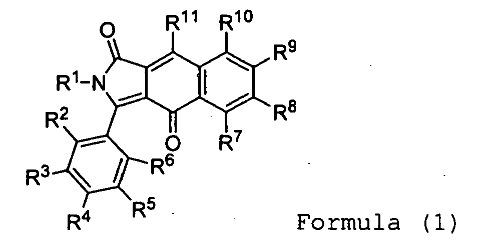 where:
R¹ denotes a hydrogen atom, an alkyl group that may have one or more substituents, or an acyl group that may have one or more substituents;
R², R³, R⁵, R⁶, R⁷, R⁸, R⁹ and R¹⁰ each independently denote a hydrogen atom, an alkyl group that may have one or more substituents, a phenyl group, a halogen atom, a cyano group, an alkoxy group that may have one or more substituents, or -NR¹²R¹³, where R¹² and R¹³ each independently denote a hydrogen atom or an alkyl group that may have one or more substituents, or R¹² and R¹³ may be bonded together to form a heterocyclic ring that may further contain a nitrogen, oxygen or sulfur atom;
R⁴ denotes a hydrogen atom, an alkyl group that may have one or more substituents, an acyl group that may have one or more substituents, or a substituent represented by Formula (2):
-Y-(Z)ₖ (2)
where Y denotes a direct bond, -O-, -S(O)ₘ- (where m denotes an integer of from 0 to 3), -CO-, -NR¹⁴-, -CONR¹⁴-, -SO₂NR¹⁴, -NR¹⁴CO-, NR¹⁴ SO₂- (where R¹⁴ denotes a hydrogen atom, an alkyl group or a hydroxylalkyl group), a linear or branched alkylene having 1 to 12 carbon atoms, or a benzene residue or triazine residue that may be substituted with an alkyl group, an amino group, a nitro group, a hydroxyl group, an alkoxy group or a halogen atom, or a linking group formed of two or more of these linking groups; Z denotes a hydrogen atom, a halogen atom, an alkyl group that may have one or more substituents, an aryl group that may have one or more substituents, a phthalimidomethyl group that may have one or more substituents, -NR¹⁵R¹⁶, -SO₃·M/n or -COO·M/n, where R¹⁵ and R¹⁶ each independently denote a hydrogen atom, an alkyl group that may have one or more substituents, an alkenyl group that may have one or more substituents or a phenyl group that may have one or more substituents, or R¹⁵ and R¹⁶ may be bonded together to form a heterocyclic ring that may further contain a nitrogen, oxygen or sulfur atom and that may have one or more substituents; M denotes a hydrogen ion, a mono- to trivalent metal ion or an ammonium ion at least one hydrogen atom of which is substituted with an alkyl group; n denotes valence of M; and k denotes an integer of 1 or 2; and
R¹¹ denotes a hydrogen atom, a halogen atom, a nitro group, a cyano group, a hydroxyl group, an alkoxy group that may have one or more substituents, an aryloxy group that may have one or more substituents, -S(O)ₘR¹⁷, -NR¹⁸R¹⁹ or -N₂⁺X⁻, where m is an integer of from 0 to 3, R¹⁷ denotes a hydrogen atom, an alkyl group that may have one or more substituents, an aryl group that may have one or more substituents, or a halogen atom; R¹⁸ and R¹⁹ each independently denote a hydrogen atom or an alkyl group that may have one or more substituents, or R¹⁸ and R¹⁹ may be bonded together to form a heterocyclic ring that may further contain a nitrogen, oxygen or sulfur atom; and X denotes a halogen atom.

5. The pigment composition according to claim 4, **characterized in that** in Formula (1):
R¹ denotes a hydrogen atom;
R², R³, R⁵, R⁶, R⁷, R⁸, R⁹ and R¹⁰ each independently denote a hydrogen atom, an alkyl group, a phenyl group, a halogen atom, a cyano group, an alkoxy group, or -NR¹²R¹³, where R¹² and R¹³ each independently denote a hydrogen atom or an alkyl group having 1 to 12 carbon atoms, or R¹² and R¹³ may be bonded together to form a heterocyclic ring that may further contain a nitrogen, oxygen or sulfur atom;
R⁴ denotes a hydrogen atom, an alkyl group that may have one or more substituents, an acyl group that may have one or more substituents, or a substituent represented by Formula (2) above; and
R¹¹ denotes a hydrogen atom, a halogen atom, a nitro group, a cyano group, a hydroxyl group, an alkoxy group, an aryloxy group, -S(O)ₘR¹⁷, -NR¹⁸R¹⁹ or -N₂⁺X⁻, where m is an integer of from 0 to 3, R¹⁷ denotes a hydrogen atom, an alkyl group, an aryl group, or a halogen atom; R¹⁸ and R¹⁹ each independently denote a hydrogen atom or an alkyl group having 1 to 12 carbon atoms, or R¹⁸ and R¹⁹ may be bonded together to form a heterocyclic ring that may further contain a nitrogen, oxygen or sulfur atom; and X denotes a halogen atom.

6. The pigment composition according to claim 5, **characterized in that** R¹¹ denotes a hydrogen atom or - NR¹⁸R¹⁹_{.}

7. The pigment composition according to claim 4, **characterized by** further comprising a pigment derivative.

8. The pigment composition according to claim 7, **characterized in that** the pigment derivative comprises at least one pigment derivative from the group consisting of pigment derivatives comprising a diketopyrrolopyrrole, a quinacridone, thiazine-indigo or anthraquinone pigment residue having a basic group, an acidic group or a phthalimidomethyl group introduced thereinto.

9. The pigment composition according to claim 4, **characterized in that** the pigment comprises a diketopyrrolopyrrole red pigment, a quinacridone red pigment, a thiazine-indigo red pigment, or an anthraquinone red pigment.

10. The pigment composition according to claim 9, **characterized in that** the pigment further comprises a yellow pigment.

11. A colored composition **characterized by** comprising a pigment composition according to claim 4, and a pigment carrier.

## Patentansprüche

1. Benzoisoindolderivat, das durch die Formel (1) dargestellt ist: 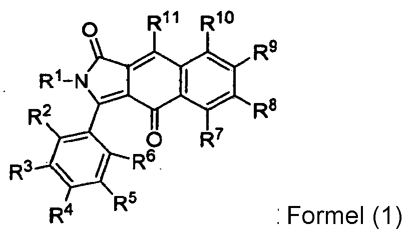 WO:
R¹ ein Wasserstoffatom, eine Alkylgruppe, die einen oder mehr Substituenten aufweisen kann, oder eine Acylgruppe, die einen oder mehr Substituenten aufweisen kann, bezeichnet;
R², R³, R⁵, R⁶, R⁷, R⁸, R⁹ und R¹⁰ jeweils unabhängig ein Wasserstoffatom, eine Alkylgruppe, die einen oder mehr Substituenten aufweisen kann, eine Phenylgruppe, ein Halogenatom, eine Cyanogruppe, eine Alkoxygruppe, die einen oder mehr Substituenten aufweisen kann, oder -NR¹2R¹³, wo R¹² und R¹³ jeweils unabhängig ein Wasserstoffatom oder eine Alkylgruppe, die einen oder mehr Substituenten aufweisen kann, bezeichnet, oder R¹² und R¹³ können zusammengebunden sein, um einen heterozyklischen Ring zu bilden, der ferner ein Stickstoff-, ein Sauerstoff- oder ein Schwefelatom enthalten kann, bezeichnet;
R⁴ ein Wasserstoffatom, eine Alkylgruppe, die einen oder mehr Substituenten aufweisen kann, eine Acylgruppe, die einen oder mehr Substituenten aufweisen kann, oder einen Substituenten, der durch die Formel (2) dargestellt ist, bezeichnet:
-Y-(Z)ₖ (2)
wo Y eine direkte Bindung, -O-, -S(O)ₘ- (wo m eine ganze Zahl von 0 bis 3 bezeichnet), -CO-, -NR¹⁴-, -CONR¹⁴-, -SO₂NR¹⁴-, NR¹⁴CO-, -NR¹⁴SO₂-(wo R¹⁴ ein Wasserstoffatom, eine Alkylgruppe oder eine Hydroxyalkylgruppe bezeichnet), ein lineares oder verzweigtes Alkylen mit 1 bis 12 Kohlenstoffatomen, oder einen Benzolrest oder einen Triazinrest, die mit einer Alkylgruppe substituiert sein können, eine Aminogruppe, eine Nitrogruppe, eine Hydroxylgruppe, eine Alkoxygruppe oder ein Halogenatom, oder eine Verbindungsgruppe, die aus zwei oder mehr von diesen Verbindungsgruppen gebildet ist, bezeichnet; Z ein Wasserstoffatom, ein Halogenatom, eine Alkylgruppe, die einen oder mehr Substituenten aufweisen kann, eine Arylgruppe, die einen oder mehrere Substituenten aufweisen kann, eine Phthalimidomethylgruppe, die einen oder mehr Substituenten aufweisen kann, -NR¹⁵R¹⁶, -SO₃·M/n oder -COO.M/n, wo R¹⁵ und R¹⁶ jeweils unabhängig ein Wasserstoffatom, eine Alkylgruppe, die einen oder mehr Substituenten aufweisen kann, eine Alkenylgruppe, die einen oder mehrere Substituenten aufweisen kann oder eine Phenylgruppe, die einen oder mehr Substituenten aufweisen kann, bezeichnet, oder R¹⁵ und R¹⁶ können zusammengebunden sein, um einen heterozyklischen Ring zu bilden, der ferner ein Stickstoff-, ein Sauerstoff- oder ein Schwefelatom enthalten kann und der einen oder mehr Substituenten aufweisen kann, bezeichnet; M ein Wasserstoffion, ein mono- bis trivalentes Metallion oder ein Ammoniumion, von welchem zumindest ein Wasserstoffatom mit einer Alkylgruppe substituiert ist, bezeichnet; n die Valenz von M bezeichnet; und k eine ganze Zahl von 1 oder 2 bezeichnet; und
R¹¹ ein Wasserstoffatom, ein Halogenatom, eine Nitrogruppe, eine Cyanogruppe, eine Hydroxylgruppe, eine Alkoxygruppe, die ein oder mehr Substituenten aufweisen kann, eine Aryloxygruppe, die einen oder mehr Substituenten aufweisen kann, -S(O)mR¹⁷, -NR¹⁸R¹⁹ oder -N₂⁺X⁻, wo m eine ganze Zahl von 0 bis 3 ist, bezeichnet, R¹⁷ ein Wasserstoffatom, eine Alkylgruppe, die einen oder mehr Substituenten aufweisen kann, eine Arylgruppe, die ein oder mehr Substituenten aufweisen kann, oder ein Halogenatom, bezeichnet; R¹⁸ und R¹⁹ jeweils unabhängig ein Wasserstoffatom oder eine Alkylgruppe, die einen oder mehr Substituenten aufweisen kann, bezeichnet, oder R¹⁸ und R¹⁹ können zusammengebunden sein, um einen heterozyklischen Ring zu bilden, der ferner ein Stickstoff-, ein Sauerstoff- oder ein Schwefelatom enthalten kann; und X ein Halogenatom bezeichnet.

2. Benzoisoindolderivat nach Anspruch 1, **dadurch gekennzeichnet, dass**:
R¹ ein Wasserstoffatom bezeichnet;
R², R³, R⁵, R⁶, R⁷ R⁸, R⁹ und R¹⁰ jeweils unabhängig ein Wasserstoffatom, eine Alkylgruppe, eine Phenylgruppe, ein Halogenatom, eine Cyanogruppe, eine Alkoxygruppe, oder -NR¹²R¹³ bezeichnet, wo R¹² und R¹³ jeweils unabhängig ein Wasserstoffatom oder eine Alkylgruppe mit 1 bis 12 Kohlenstoffatomen bezeichnet, oder R¹² und R¹³ können zusammengebunden sein, um einen heterozyklischen Ring zu bilden, der ferner ein Stickstoff-, ein Sauerstoff- oder ein Schwefelatom enthalten kann;
R⁴ ein Wasserstoffatom, eine Alkylgruppe, die einen oder mehr Substituenten aufweisen kann, eine Acylgruppe, die einen oder mehr Substituenten aufweisen kann, oder einen Substituenten, der durch die obige Formel (2) dargestellt ist, bezeichnet; und
R¹¹ ein Wasserstoffatom, ein Halogenatom, eine Nitrogruppe, eine Cyanogruppe, eine Hydroxylgruppe, eine Alkoxygruppe, eine Aryloxygruppe, -S(O)ₘR¹⁷, -NR¹⁸R¹⁹ oder -N₂⁺X⁻ bezeichnet, wo m eine ganze Zahl von 0 bis 3 ist, R¹⁷ ein Wasserstoffatom, eine Alkylgruppe, eine Arylgruppe oder ein Halogenatom bezeichnet; R¹⁸ und R¹⁹ jeweils unabhängig ein Wasserstoffatom oder eine Alkylgruppe mit 1 bis 12 Kohlenstoffatomen bezeichnet, oder R¹⁸ und R¹⁹ können zusammen gebunden sein, um einen heterozyklischen Ring zu bilden, der ferner ein Stickstoff-, ein Sauerstoff- oder ein Schwefelatom enthalten kann; und X ein Halogenatom bezeichnet.

3. Benzoisoindolderivat nach Anspruch 2, **dadurch gekennzeichnet, dass** R¹¹ ein Wasserstoffatom oder -NR¹⁸R¹⁹ bezeichnet.

4. Pigmentzusammensetzung, die ein Pigment und ein Benzoisoindolderivat, das durch die Formel (1) dargestellt ist, umfasst: 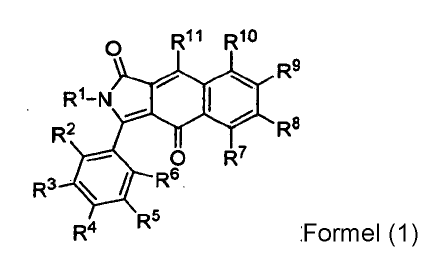 WO:
R¹ ein Wasserstoffatom, eine Alkylgruppe, die einen oder mehr Substituenten aufweisen kann, oder eine Acylgruppe, die einen oder mehr Substituenten aufweisen kann, bezeichnet;
R², R³, R⁵, R⁶, R⁷, R⁸, R⁹ und R¹⁰ jeweils unabhängig ein Wasserstoffatom, eine Alkylgruppe, die einen oder mehr Substituenten aufweisen kann, eine Phenylgruppe, ein Halogenatom, eine Cyanogruppe, eine Alkoxygruppe, die einen oder mehr Substituenten aufweisen kann, oder -NR¹2R¹³, wo R¹² und R¹³ jeweils unabhängig ein Wasserstoffatom oder eine Alkylgruppe, die einen oder mehr Substituenten aufweisen kann, bezeichnet, oder R¹² und R¹³ können zusammengebunden sein, um einen heterozyklischen Ring zu bilden, der ferner ein Stickstoff-, ein Sauerstoff- oder ein Schwefelatom enthalten kann, bezeichnet;
R⁴ ein Wasserstoffatom, eine Alkylgruppe, die einen oder mehr Substituenten aufweisen kann, eine Acylgruppe, die einen oder mehr Substituenten aufweisen kann, oder einen Substituenten, der durch die Formel (2) dargestellt ist, bezeichnet:
-Y-(Z)ₖ (2)
wo Y eine direkte Bindung, -O-, -S(O)ₘ- (wo m eine ganze Zahl von 0 bis 3 bezeichnet), -CO-, -NR¹⁴-, -CONR¹⁴-, -SO₂NR¹⁴-, NR¹⁴CO-, -NR¹⁴SO₂-(wo R¹⁴ ein Wasserstoffatom, eine Alkylgruppe oder eine Hydroxyalkylgruppe bezeichnet), ein lineares oder verzweigtes Alkylen mit 1 bis 12 Kohlenstoffatomen, oder einen Benzolrest oder einen Triazinrest, die mit einer Alkylgruppe substituiert sein können, eine Aminogruppe, eine Nitrogruppe, eine Hydroxylgruppe, eine Alkoxygruppe oder ein Halogenatom, oder eine Verbindungsgruppe, die aus zwei oder mehr von diesen Verbindungsgruppen gebildet ist, bezeichnet; Z ein Wasserstoffatom, ein Halogenatom, eine Alkylgruppe, die einen oder mehr Substituenten aufweisen kann, eine Arylgruppe, die einen oder mehrere Substituenten aufweisen kann, eine Phthalimidomethylgruppe, die einen oder mehr Substituenten aufweisen kann, -NR¹⁵R¹⁶, -SO₃·M/n oder -COO·M/n, wo R¹⁵ und R¹⁶ jeweils unabhängig ein Wasserstoffatom, eine Alkylgruppe, die einen oder mehr Substituenten aufweisen kann, eine Alkenylgruppe, die einen oder mehrere Substituenten aufweisen kann oder eine Phenylgruppe, die einen oder mehr Substituenten aufweisen kann, bezeichnet, oder R¹⁵ und R¹⁶ können zusammengebunden sein, um einen heterozyklischen Ring zu bilden, der ferner ein Stickstoff-, ein Sauerstoff- oder ein Schwefelatom enthalten kann und der einen oder mehr Substituenten aufweisen kann, bezeichnet; M ein Wasserstoffion, ein mono- bis trivalentes Metallion oder ein Ammoniumion, von welchem zumindest ein Wasserstoffatom mit einer Alkylgruppe substituiert ist, bezeichnet; n die Valenz von M bezeichnet; und k eine ganze Zahl von 1 oder 2 bezeichnet; und
R¹¹ ein Wasserstoffatom, ein Halogenatom, eine Nitrogruppe, eine Cyanogruppe, eine Hydroxylgruppe, eine Alkoxygruppe, die einen oder mehr Substituenten aufweisen kann, eine Aryloxygruppe, die einen oder mehr Substituenten aufweisen kann, -S(O)mR¹⁷, -NR¹⁸R¹⁹ oder -N₂⁺X⁻, wo m eine ganze Zahl von 0 bis 3 ist, bezeichnet, R¹⁷ ein Wasserstoffatom, eine Alkylgruppe, die einen oder mehr Substituenten aufweisen kann, eine Arylgruppe, die einen oder mehr Substituenten aufweisen kann, oder ein Halogenatom, bezeichnet; R¹⁸ und R¹⁹ jeweils unabhängig ein Wasserstoffatom oder eine Alkylgruppe, die einen oder mehr Substituenten aufweisen kann, bezeichnet, oder R¹⁸ und R¹⁹ können zusammengebunden sein, um einen heterozyklischen Ring zu bilden, der ferner ein Stickstoff-, ein Sauerstoff- oder ein Schwefelatom enthalten kann; und X ein Halogenatom bezeichnet.

5. Pigmentzusammensetzung nach Anspruch 4, **dadurch gekennzeichnet, dass** in Formel (1):
R¹ ein Wasserstoffatom bezeichnet;
R², R³, R⁵, R⁶, R⁷, R⁸, R⁹ und R¹⁰ jeweils unabhängig ein Wasserstoffatom, eine Alkylgruppe, eine Phenylgruppe, ein Halogenatom, eine Cyanogruppe, eine Alkoxygruppe, oder -NR¹²R¹³ bezeichnet, wo R¹² und R¹³ jeweils unabhängig ein Wasserstoffatom oder eine Alkylgruppe mit 1 bis 12 Kohlenstoffatomen bezeichnet, oder R¹² und R¹³ können zusammengebunden sein, um einen heterozyklischen Ring zu bilden, der ferner ein Stickstoff-, ein Sauerstoff- oder ein Schwefelatom enthalten kann;
R⁴ ein Wasserstoffatom, eine Alkylgruppe, die einen oder mehr Substituenten aufweisen kann, eine Acylgruppe, die einen oder mehr Substituenten aufweisen kann, oder einen Substituenten, der durch die obige Formel (2) dargestellt ist, bezeichnet; und
R¹¹ ein Wasserstoffatom, ein Halogenatom, eine Nitrogruppe, eine Cyanogruppe, eine Hydroxylgruppe, eine Alkoxygruppe, eine Aryloxygruppe, -S(O)ₘR¹⁷, -NR¹⁸R¹⁹ oder -N2⁺X⁻ bezeichnet, wo m eine ganze Zahl von 0 bis 3 ist, R¹⁷ ein Wasserstoffatom, eine Alkylgruppe, eine Arylgruppe oder ein Halogenatom bezeichnet; R¹⁸ und R¹⁹ jeweils unabhängig ein Wasserstoffatom oder eine Alkylgruppe mit 1 bis 12 Kohlenstoffatomen bezeichnet, oder R¹⁸ und R¹⁹ können zusammen gebunden sein, um einen heterozyklischen Ring zu bilden, der ferner ein Stickstoff-, ein Sauerstoff- oder ein Schwefelatom enthalten kann; und X ein Halogenatom bezeichnet.

6. Pigmentzusammensetzung nach Anspruch 5, **dadurch gekennzeichnet, dass** R¹¹ ein Wasserstoffatom oder -NR¹⁸R¹⁹ bezeichnet.

7. Pigmentzusammensetzung nach Anspruch 4, **gekennzeichnet durch** ferner Umfassen eines Pigmentderivats.

8. Pigmentzusammensetzung nach Anspruch 7, **dadurch gekennzeichnet, dass** das Pigmentderivat zumindest ein Pigmentderivat von der Gruppe umfasst, die aus Pigmentderivaten besteht, die ein Diketopyrrolopyrrol, ein Quinacridon, Thiazin-Indigo- oder Anthraquinonpigmentreste mit einer darin eingeführten basischen Gruppe, einer sauren Gruppe oder einer Phthalimidomethylgruppe, umfasst.

9. Pigmentzusammenfassung nach Anspruch 4, **dadurch gekennzeichnet, dass** das Pigment ein Diketopyrrolopyrrolrotpigment, ein Quinacridonrotpigment, ein Thiazin-Indigo-Rotpigment oder ein Anthraquinonrotpigment umfasst.

10. Pigmentzusammensetzung nach Anspruch 9, **dadurch gekennzeichnet, dass** das Pigment ferner ein gelbes Pigment umfasst.

11. Gefärbte Zusammensetzung, die **gekennzeichnet ist durch** Umfassen einer Pigmentzusammensetzung nach Anspruch 4, und eines Pigmentträgers.

## Revendications

1. Dérivé de benzoisoindole représenté par la Formule (1) : 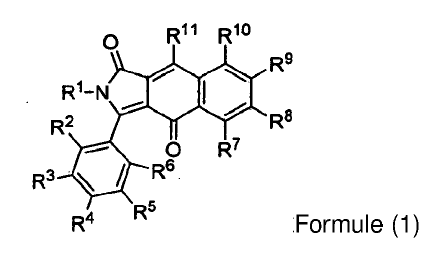 dans laquelle :
R¹ représente un atome d'hydrogène, un groupe alkyle qui peut comporter un ou plusieurs substituants, ou un groupe acyle qui peut comporter un ou plusieurs substituants ;
R², R³, R⁵, R⁶, R⁷, R⁸, R⁹ et R¹⁰ représentent chacun indépendamment un atome d'hydrogène, un groupe alkyle qui peut comporter un ou plusieurs substituants, un groupe phényle, un atome d'halogène, un groupe cyano, un groupe alcoxy qui peut comporter un ou plusieurs substituants, ou un groupe - NR¹²R¹³ dans lequel les radicaux R¹² et R¹³ représentent chacun indépendamment un atome d'hydrogène ou un groupe alkyle qui peut comporter un ou plusieurs substituants, ou R¹² et R¹³ peuvent être reliés l'un à l'autre pour former un noyau hétérocyclique qui peut en outre contenir un atome d'azote, d'oxygène ou de soufre ;
R⁴ représente un atome d'hydrogène, un groupe alkyle qui peut comporter un ou plusieurs substituants, un groupe acyle qui peut comporter un ou plusieurs substituants, ou un substituant représenté par la Formule (2) :
-Y-(Z)ₖ (2)
dans laquelle Y représente une liaison directe, un groupe -O-, un groupe - S(O)ₘ- (dans lequel m représente un nombre entier de 0 à 3), un groupe -CO-, un groupe -NR¹⁴-, un groupe -CONR¹⁴-, un groupe -SO₂NR¹⁴, un groupe -NR¹⁴CO-, un groupe NR¹⁴SO₂- (groupes dans lesquels R¹⁴ représente un atome d'hydrogène, un groupe alkyle ou un groupe hydroxylalkyle), un groupe alkylène linéaire ou ramifié comportant de 1 à 12 atomes de carbone, ou un résidu de benzène ou un résidu de triazine qui peut être substitué par un groupe alkyle, un groupe amino, un groupe nitro, un groupe hydroxyle, un groupe alcoxy ou un atome d'halogène, ou un groupe de liaison constitué de deux ou plusieurs de ces groupes de liaison ; Z représente un atome d'hydrogène, un atome d'halogène, un groupe alkyle qui peut comporter un ou plusieurs substituants, un groupe aryle qui peut comporter un ou plusieurs substituants, un groupe phtalimidométhyle qui peut comporter un ou plusieurs substituants, un groupe -NR¹⁵R¹⁶, un groupe - SO₃-M/n ou un groupe -COO-M/n, où R¹⁵ et R¹⁶ représentent chacun indépendamment un atome d'hydrogène, un groupe alkyle qui peut comporter un ou plusieurs substituants, un groupe alcényle qui peut comporter un ou plusieurs substituants ou un groupe phényle qui peut comporter un ou plusieurs substituants, ou R¹⁵ et R¹⁶ peuvent être reliés l'un à l'autre pour former un noyau hétérocyclique qui peut en outre contenir un atome d'azote, d'oxygène ou de soufre et qui peut comporter un ou plusieurs substituants ; M représente un ion d'hydrogène, un ion de métal mono- à trivalent ou un ion d'ammonium dont au moins un atome d'hydrogène est substitué par un groupe alkyle ; n représente la valence de M ; et k représente un nombre entier de 1 ou 2 ; et
R¹¹ représente un atome d'hydrogène, un atome d'halogène, un groupe nitro, un groupe cyano, un groupe hydroxyle, un groupe alcoxy qui peut comporter un ou plusieurs substituants, un groupe aryloxy qui peut comporter un ou plusieurs substituants, un groupe -S(O)ₘR¹⁷, un groupe -NR¹⁸R¹⁹ ou un groupe -N₂⁺X⁻, groupes dans lesquels m représente un nombre entier de 0 à 3, R¹⁷ représente un atome d'hydrogène, un groupe alkyle qui peut comporter un ou plusieurs substituants, un groupe aryle qui peut comporter un ou plusieurs substituants, ou un atome d'halogène ; R¹⁸ et R¹⁹ représentent chacun indépendamment un atome d'hydrogène ou un groupe alkyle qui peut comporter un ou plusieurs substituants, ou R¹⁸ et R¹⁹ peuvent être reliés l'un à l'autre pour former un noyau hétérocyclique qui peut en outre contenir un atome d'azote, d'oxygène ou de soufre ; et X représente un atome d'halogène.

2. Dérivé de benzoisoindole selon la revendication 1, **caractérisé en ce que** :
R¹ représente un atome d'hydrogène ;
R², R³, R⁵, R⁶, R⁷, R⁸, R⁹ et R¹⁰ représentent chacun indépendamment un atome d'hydrogène, un groupe alkyle, un groupe phényle, un atome d'halogène, un groupe cyano, un groupe alcoxy, ou un groupe -NR¹²R¹³ dans lequel les radicaux R¹² et R¹³ représentent chacun indépendamment un atome d'hydrogène ou un groupe alkyle comportant de 1 à 12 atomes de carbone, ou R¹² et R¹³ peuvent être reliés l'un à l'autre pour former un noyau hétérocyclique qui peut en outre contenir un atome d'azote, d'oxygène ou de soufre ;
R⁴ représente un atome d'hydrogène, un groupe alkyle qui peut comporter un ou plusieurs substituants, un groupe acyle qui peut comporter un ou plusieurs substituants, ou un substituant représenté par la Formule (2) ci-dessus ; et
R¹¹ représente un atome d'hydrogène, un atome d'halogène, un groupe nitro, un groupe cyano, un groupe hydroxyle, un groupe alcoxy, un groupe aryloxy, un groupe -S(O)ₘR¹⁷, un groupe -NR¹⁸R¹⁹ ou un groupe -N₂⁺X⁻, groupes dans lesquels m représente un nombre entier de 0 à 3, R¹⁷ représente un atome d'hydrogène, un groupe alkyle, un groupe aryle, ou un atome d'halogène ; R¹⁸ et R¹⁹ représentent chacun indépendamment un atome d'hydrogène ou un groupe alkyle comportant de 1 à 12 atomes de carbone, ou R¹⁸ et R¹⁹ peuvent être reliés l'un à l'autre pour former un noyau hétérocyclique qui peut en outre contenir un atome d'azote, d'oxygène ou de soufre ; et X représente un atome d'halogène.

3. Dérivé de benzoisoindole selon la revendication 2, **caractérisé en ce que** R¹¹ représente un atome d'hydrogène ou un groupe -NR¹⁸R¹⁹.

4. Composition de pigment comportant un pigment et un dérivé de benzoisoindole représenté par la Formule (1) : 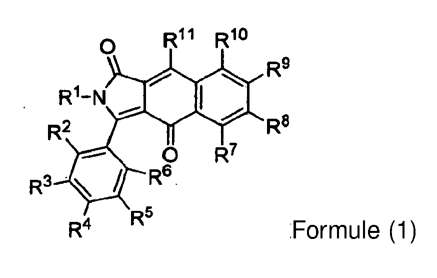 dans laquelle :
R¹ représente un atome d'hydrogène, un groupe alkyle qui peut comporter un ou plusieurs substituants, ou un groupe acyle qui peut comporter un ou plusieurs substituants ;
R², R³, R⁵, R⁶, R⁷, R⁸, R⁹ et R¹⁰ représentent chacun indépendamment un atome d'hydrogène, un groupe alkyle qui peut comporter un ou plusieurs substituants, un groupe phényle, un atome d'halogène, un groupe cyano, un groupe alcoxy qui peut comporter un ou plusieurs substituants, ou un groupe - NR¹²R¹³ dans lequel les radicaux R¹² et R¹³ représentent chacun indépendamment un atome d'hydrogène ou un groupe alkyle qui peut comporter un ou plusieurs substituants, ou R¹² et R¹³ peuvent être reliés l'un à l'autre pour former un noyau hétérocyclique qui peut en outre contenir un atome d'azote, d'oxygène ou de soufre ;
R⁴ représente un atome d'hydrogène, un groupe alkyle qui peut comporter un ou plusieurs substituants, un groupe acyle qui peut comporter un ou plusieurs substituants, ou un substituant représenté par la Formule (2) :
-Y-(Z)ₖ (2)
dans laquelle Y représente une liaison directe, un groupe -O-, un groupe - S(O)ₘ- (dans lequel m représente un nombre entier de 0 à 3), un groupe -CO-, un groupe -NR¹⁴-, un groupe -CONR¹⁴-, un groupe -SO₂NR¹⁴, un groupe -NR¹⁴CO-, un groupe NR¹⁴SO₂- (groupes dans lesquels R¹⁴ représente un atome d'hydrogène, un groupe alkyle ou un groupe hydroxylalkyle), un groupe alkylène linéaire ou ramifié comportant de 1 à 12 atomes de carbone, ou un résidu de benzène ou un résidu de triazine qui peut être substitué par un groupe alkyle, un groupe amino, un groupe nitro, un groupe hydroxyle, un groupe alcoxy ou un atome d'halogène, ou un groupe de liaison constitué de deux ou plusieurs de ces groupes de liaison ; Z représente un atome d'hydrogène, un atome d'halogène, un groupe alkyle qui peut comporter un ou plusieurs substituants, un groupe aryle qui peut comporter un ou plusieurs substituants, un groupe phtalimidométhyle qui peut comporter un ou plusieurs substituants, un groupe -NR¹⁵R¹⁶, un groupe - SO₃-M/n ou un groupe -COO-M/n, où R¹⁵ et R¹⁶ représentent chacun indépendamment un atome d'hydrogène, un groupe alkyle qui peut comporter un ou plusieurs substituants, un groupe alcényle qui peut comporter un ou plusieurs substituants ou un groupe phényle qui peut comporter un ou plusieurs substituants, ou R¹⁵ et R¹⁶ peuvent être reliés l'un à l'autre pour former un noyau hétérocyclique qui peut en outre contenir un atome d'azote, d'oxygène ou de soufre et qui peut comporter un ou plusieurs substituants ; M représente un ion d'hydrogène, un ion de métal mono- à trivalent ou un ion d'ammonium dont au moins un atome d'hydrogène est substitué par un groupe alkyle ; n représente la valence de M ; et k représente un nombre entier de 1 ou 2 ; et
R¹¹ représente un atome d'hydrogène, un atome d'halogène, un groupe nitro, un groupe cyano, un groupe hydroxyle, un groupe alcoxy qui peut comporter un ou plusieurs substituants, un groupe aryloxy qui peut comporter un ou plusieurs substituants, un groupe -S(O)ₘR¹⁷, un groupe -NR¹⁸R¹⁹ ou un groupe -N₂⁺X⁻, groupes dans lesquels m représente un nombre entier de 0 à 3, R¹⁷ représente un atome d'hydrogène, un groupe alkyle qui peut comporter un ou plusieurs substituants, un groupe aryle qui peut comporter un ou plusieurs substituants, ou un atome d'halogène ; R¹⁸ et R¹⁹ représentent chacun indépendamment un atome d'hydrogène ou un groupe alkyle qui peut comporter un ou plusieurs substituants, ou R¹⁸ et R¹⁹ peuvent être reliés l'un à l'autre pour former un noyau hétérocyclique qui peut en outre contenir un atome d'azote, d'oxygène ou de soufre ; et X représente un atome d'halogène.

5. Composition de pigment selon la revendication 4, **caractérisée en ce que** dans la Formule (1) :
R¹ représente un atome d'hydrogène ;
R², R³, R⁵, R⁶, R⁷, R⁸, R⁹ et R¹⁰ représentent chacun indépendamment un atome d'hydrogène, un groupe alkyle, un groupe phényle, un atome d'halogène, un groupe cyano, un groupe alcoxy, ou un groupe -NR¹²R¹³ dans lequel les radicaux R¹² et R¹³ représentent chacun indépendamment un atome d'hydrogène ou un groupe alkyle comportant de 1 à 12 atomes de carbone, ou R¹² et R¹³ peuvent être reliés l'un à l'autre pour former un noyau hétérocyclique qui peut en outre contenir un atome d'azote, d'oxygène ou de soufre ;
R⁴ représente un atome d'hydrogène, un groupe alkyle qui peut comporter un ou plusieurs substituants, un groupe acyle qui peut comporter un ou plusieurs substituants, ou un substituant représenté par la Formule (2) ci-dessus ; et
R¹¹ représente un atome d'hydrogène, un atome d'halogène, un groupe nitro, un groupe cyano, un groupe hydroxyle, un groupe alcoxy, un groupe aryloxy, un groupe -S(O)ₘR¹⁷, un groupe -NR¹⁸R¹⁹ ou un groupe -N₂⁺X⁻, groupes dans lesquels m représente un nombre entier de 0 à 3, R¹⁷ représente un atome d'hydrogène, un groupe alkyle, un groupe aryle, ou un atome d'halogène ; R¹⁸ et R¹⁹ représentent chacun indépendamment un atome d'hydrogène ou un groupe alkyle comportant de 1 à 12 atomes de carbone, ou R¹⁸ et R¹⁹ peuvent être reliés l'un à l'autre pour former un noyau hétérocyclique qui peut en outre contenir un atome d'azote, d'oxygène ou de soufre ; et X représente un atome d'halogène.

6. Composition de pigment selon la revendication 5, **caractérisée en ce que** R¹¹ représente un atome d'hydrogène ou un groupe -NR¹⁸R¹⁹.

7. Composition de pigment selon la revendication 4, **caractérisée en ce qu'**elle comporte en outre un dérivé de pigment.

8. Composition de pigment selon la revendication 7, **caractérisée en ce que** le dérivé de pigment comporte au moins un dérivé de pigment du groupe constitué des dérivés de pigment comportant un résidu de pigment de type dicétopyrrolopyrrole, quinacridone, thiazine-indigo ou anthraquinone dans lequel est introduit un groupe basique, un groupe acide ou un groupe phtalimidométhyle.

9. Composition de pigment selon la revendication 4, **caractérisée en ce que** le pigment comporte un pigment rouge de dicétopyrrolopyrrole, un pigment rouge de quinacridone, un pigment rouge de thiazine-indigo ou un pigment rouge d'anthraquinone.

10. Composition de pigment selon la revendication 9, **caractérisée en ce que** le pigment comporte en outre un pigment jaune.

11. Composition colorée **caractérisée en ce qu'**elle comporte une composition de pigment selon la revendication 4, et un support de pigment.
